# EUROPEAN PATENT APPLICATION

(11) **EP 4 793 275 A1**
(43) Date of publication of application: **19.08.2026**
(21) Application number: 24876586.9
(22) Date of filing: 10.10.2024
(51) Int. Cl.: C07D 403/14, C07D 405/14, C07D 409/14, C07D 413/14, C07D 417/14, A61K 31/501, A61P 25/00, A61P 25/04

(54) **AT2R ANTAGONIST AND USE THEREOF**

(30) Priority: 10.10.2023 CN 202311307596; 28.02.2024 CN 202410224525; 26.09.2024 CN 202411356101
(71) Applicant: Hubei Bio-Pharmaceutical Industrial Technological Institute Inc., Wuhan, Hubei 430075 (CN)
(72) Inventor: ZHANG, Xuejun, Wuhan, Hubei 430075 (CN); ZANG, Yang, Wuhan, Hubei 430075 (CN); ZHUO, Junming, Wuhan, Hubei 430075 (CN); SUN, Xiaochuan, Wuhan, Hubei 430075 (CN); FU, Haoliang, Wuhan, Hubei 430075 (CN); AN, Ke, Wuhan, Hubei 430075 (CN); DENG, Jianwen, Wuhan, Hubei 430075 (CN)
(74) Representative: Dehns
(86) International application number: PCT/CN2024/123916
(87) International publication number: WO 2025/077774

(57) **Abstract**

The present invention provides a compound as shown in formula I. The compound can be used as an AT2R antagonist.

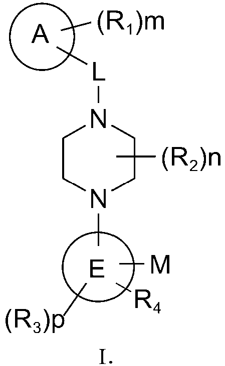

## Description

### PRIORITY INFORMATION

This application claims priority to:
Chinese Patent Application No. 202311307596.5, titled "AT2R ANTAGONIST AND USE THEREOF" and filed with China National Intellectual Property Administration on October 10, 2023;
Chinese Patent Application No. 202410224525.7, titled "AT2R ANTAGONIST AND USE THEREOF" and filed with China National Intellectual Property Administration on February 28, 2024; and
Chinese Patent Application No. 202411356101.2, titled "AT2R ANTAGONIST AND USE THEREOF" and filed with China National Intellectual Property Administration on September 26, 2024,
the entire disclosures of which are incorporated herein by reference.

### FIELD

The present disclosure belongs to the pharmaceutical field. Particularly, the present disclosure relates to a heterocyclic compound as an angiotensin II (Ang II) type 2 receptor (AT2R) antagonist and use thereof.

### BACKGROUND

Neuropathic pain is a chronic pain disease caused by primary injury or dysfunction of a nervous system, which may be classified into peripheral neuropathic pain and central neuropathic pain based on a lesion site. The neuropathic pain may be induced by trauma, inflammation, infection, or compression, including conditions such as diabetic neuropathic pain (DNP), postherpetic neuralgia (PHN), primary neuropathy, secondary neuropathy, peripheral neuropathy, and neuropathy caused by mechanical nerve injury or biochemical nerve injury. Currently, drugs used clinically to treat the neuropathic pain mainly include antiepileptic drugs, antidepressants, and opioid analgesics, such as gabapentin, pregabalin, and tricyclic antidepressants. However, these drugs lack specificity, provide very limited therapeutic efficacy, and are associated with severe side effects, including cognitive changes, sedation, nausea, as well as tolerance and dependence, and thus falling far short of clinical drug needs. Therefore, it is imperative to study pathogenesis of the neuropathic pain, identify clearly defined drug targets, and develop novel drugs that can effectively treat the neuropathic pain with few adverse reactions.

In humans, two major types of angiotensin II (Ang II) receptors have been identified, designated as Ang II type 1 receptor (AT1R) and Ang II type 2 receptor (AT2R). Ang II has exhibited functions of regulating blood pressure, and fluid and electrolyte homeostasis in numerous organs, including kidneys, adrenal glands, heart, blood vessels, brain, gastrointestinal tract, and reproductive organs. An effect of Ang II is regulated by a balance of an expression of two G protein-coupled receptors (GPCRs), including AT1R and AT2R. AT1R is expressed throughout an entire life cycle and is primarily responsible for regulating the blood pressure. Its blockers are widely used clinically as antihypertensive agents. AT1R can mediate most of physiological effects of Ang II. AT2R is primarily expressed in embryonic tissues and is related to blood pressure regulation, nerve growth, pain control, and myocardial regeneration. Drugs targeting AT2R can improve cardiovascular function and alleviate the neuropathic pain. AT2R is implicated in pain mechanisms in the nervous system and is primarily expressed in dorsal root ganglia (DRG) and trigeminal ganglia. Compared with normal nerves, damaged nerves and painful neuromas exhibit higher levels of AT2R expression. AT2R, after activation, can sensitize ion channels in neurons via second messenger pathways activated by the G protein-coupled receptors. Sensitization can lead to the activation of the ion channels, thereby inducing neuronal excitation. AT2R antagonists have been proven to be applicable for analgesia through animal experiments and clinical trials.

Despite notable advancements in the research and application of the AT2R antagonists, there is still substantial room for improvement, necessitating ongoing research and development of novel AT2R antagonists.

### SUMMARY

An object of the present disclosure is to provide a compound as an AT2R antagonist and use thereof. The compound has a structure represented in a first aspect of the present disclosure. The compound can be used to prepare a medicament, a pharmaceutical composition, or a formulation for treating and/or preventing an AT2R-related disease or disorder; or can be used to treat and/or prevent an AT2R-related disease or disorder.

In a first aspect of the present disclosure, there is provided a compound represented by Formula I, or a tautomer, stereoisomer, or pharmaceutically acceptable salt thereof: 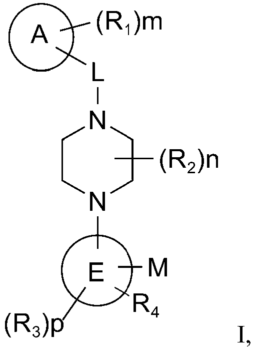 wherein:
ring A is selected from a 5- to 6-membered heterocyclic ring;
R₁ is selected from halogen, hydroxyl, amino, cyano, oxo (=O), C₁ to C₆ alkyl, C₁ to C₆ alkoxy, and C₃ to C₇ cycloalkyl, wherein the C₁ to C₆ alkyl, the C₁ to C₆ alkoxy, and the C₃ to C₇ cycloalkyl are optionally substituted with one to four substituents selected from halogen, hydroxyl, amino, cyano, C₁ to C₆ alkyl, C₁ to C₆ alkoxy, C₃ to C₇ cycloalkyl, and oxo;
m is selected from 0, 1, 2, 3, or 4, and when a plurality of substituents R₁ are present, the plurality of substituents R₁ are the same or different;
L is absent or selected from C₀ to C₆ alkyl-O-C₀ to C₆ alkyl, C₀ to C₆ alkyl-NH-Co to C₆ alkyl, and C₁ to C₆ alkyl, wherein the C₁ to C₆ alkyl-O-C₁ to C₆ alkyl, the C₀ to C₆ alkyl-NH-Co to C₆ alkyl, and the C₁ to C₆ alkyl are optionally substituted with one to four substituents selected from deuterium, oxo (=O), methyl, ethyl, halogenated methyl, and halogenated ethyl;
R₂ is selected from oxo (=O), C₁ to C₆ alkyl, C₃ to C₇ cycloalkyl, a 5- to 10-membered heterocyclic ring, and a 6- to 10-membered aromatic ring, wherein the C₁ to C₆ alkyl, the C₃ to C₆ cycloalkyl, the 5- to 10-membered heterocyclic ring, and the 6- to 10-membered aromatic ring are optionally substituted with one, two, three, or four substituents selected from halogen, hydroxyl, cyano, amino, C₁ to C₆ alkyl, C₁ to C₆ alkoxy, and C₃ to C₇ cycloalkyl;
n is selected from 0, 1, 2, 3, 4, 5, 6, 7, or 8, and when a plurality of substituents R₂ are present, the plurality of substituents R₂ are the same or different;
ring E is selected from a benzene ring or a 5- to 6-membered heteroaromatic ring;
R₃ is selected from halogen, hydroxyl, cyano, dimethylamino, C₁ to C₆ alkyl, C₁ to C₆ alkoxy, and C₃ to C₇ cycloalkyl, wherein the C₁ to C₆ alkyl, the C₁ to C₆ alkoxy, and the C₃ to C₇ cycloalkyl are optionally substituted with one to four substituents selected from halogen, hydroxyl, cyano, C₁ to C₆ alkyl, C₁ to C₆ alkoxy, and C₃ to C₇ cycloalkyl;
p is selected from 0, 1, 2, 3, or 4, and when a plurality of substituents R₃ are present, the plurality of substituents R₃ are the same or different;
M is selected from carboxylic group, -CONHSO₂R₅, -SO₂NHCOR₅, tetrazole, or a tetrazole bioisostere, wherein the tetrazole bioisostere includes, but is not limited to, phosphoric acid, 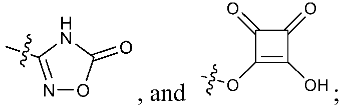
R₄ is selected from -CHF₂, -CH₂F, -C₀ to C₆ alkyl-O-halogenated C₁ to C₆ alkyl and -C₀ to C₆ alkyl-O-C₃ to C₄ cycloalkyl, wherein the -Co to C₆ alkyl-O-halogenated C₁ to C₆ alkyl and the -C₀ to C₆ alkyl-O-C₃ to C₄ cycloalkyl are optionally substituted with one to four substituents selected from hydroxyl, amino, C₃ to C₇ heterocycloalkyl, C₁ to C₃ alkyl, C₃ to C₆ cycloalkyl, and C₁ to C₃ alkoxy;
M and R₄ are on the ring E in an ortho relationship; and
R₅ is selected from C₁ to C₈ alkyl, C₃ to C₈ cycloalkyl, C₁ to C₈ alkoxy, and a 5- to 6-membered heterocyclic ring, wherein the C₁ to C₈ alkyl, the C₃ to C₈ cycloalkyl, the C₁ to C₈ alkoxy, and the 5- to 6-membered heterocyclic ring are optionally substituted with one to four substituents selected from halogen, amino, C₃ to C₇ heterocycloalkyl, C₁ to C₃ alkyl, C₃ to C₆ cycloalkyl, and C₁ to C₃ alkoxy.

In a preferred embodiment of the present disclosure, the ring A is a 5- to 6-membered heteroaromatic ring, 5- to 6-membered heterocycloalkenyl, or 5- to 6-membered heterocycloalkyl, wherein the 5- to 6-membered heteroaromatic ring, the 5- to 6-membered heterocycloalkenyl, or the 5- to 6-membered heterocycloalkyl includes one, two, or three identical or different heteroatoms, wherein the one, two, or three identical or different heteroatoms are selected from N, O, and S.

In a preferred embodiment of the present disclosure, the ring A is selected from: furanyl, pyrrolyl, thienyl, oxazolyl, oxadiazolyl, thiazolyl, thiadiazolyl, pyrazolyl, imidazolyl, triazolyl, tetrazolyl, pyridinyl, pyrimidinyl, pyridazinyl, triazinyl, dihydropyrrolyl, dihydropyrazolyl, dihydroimidazolyl, dihydrotriazolyl, dihydrotetrazolyl, dihydrotriazolyl, dihydrotetrazolyl, tetrahydropyridinyl, dihydropyridinyl, tetrahydropyrazinyl, dihydropyrazinyl, tetrahydropyrimidinyl, dihydropyrimidinyl, pyrrolidinyl, pyrazolidinyl, imidazolidinyl, piperidinyl, piperazinyl, hexahydropyridazinyl, and hexahydropyrimidinyl.

In the present disclosure, unless otherwise specified, when m, n, and p are zero, a group on a carbon atom to which R₁, R₂, and R₃ is bonded is H.

In a preferred embodiment of the present disclosure, the ring A is selected from pyridinyl, pyrimidinyl, pyridazinyl, pyrazinyl, and thiadiazolyl.

In a preferred embodiment of the present disclosure, the ring E is a benzene ring or a 5- to 6-membered heteroaromatic ring, wherein the 5- to 6-membered heteroaromatic ring includes one, two, or three identical or different heteroatoms, wherein the one, two, or three identical or different heteroatoms are selected from N, O, and S.

In a preferred embodiment of the present disclosure, the ring E is selected from a benzene ring, pyridine, pyrimidine, and pyridazine.

In a preferred embodiment of the present disclosure, the ring E is selected from a benzene ring.

In a preferred embodiment of the present disclosure, M and R₄ are on the ring E in an ortho relationship.

In a preferred embodiment of the present disclosure, R₁ is selected from halogen, hydroxyl, amino, cyano, oxo (=O), C₁ to C₃ alkyl, halogenated C₁ to C₃ alkyl, C₁ to C₃ alkoxy, halogenated C₁ to C₃ alkoxy, C₃ to C₇ cycloalkyl, C₁ to C₃ alkyl-C₁ to C₃ alkoxy, and C₁ to C₃ alkyl-C₃ to C₇ cycloalkyl.

In a preferred embodiment of the present disclosure, R₁ is selected from halogen, methyl, halogenated methyl, and methoxy.

In a preferred embodiment of the present disclosure, m is selected from 0, 1, 2, 3, or 4.

In a preferred embodiment of the present disclosure, m is selected from 0 or 1.

In a preferred embodiment of the present disclosure, R₁ is selected from halogen, hydroxyl, amino, cyano, oxo (=O), C₁ to C₃ alkyl, halogenated C₁ to C₃ alkyl, C₁ to C₃ alkoxy, halogenated C₁ to C₃ alkoxy, C₃ to C₇ cycloalkyl, C₁ to C₃ alkyl-C₁ to C₃ alkoxy, and C₁ to C₃ alkyl-C₃ to C₇ cycloalkyl, and m is selected from 0, 1, 2, 3, or 4.

In a preferred embodiment of the present disclosure, R₁ is selected from halogen, methyl, halogenated methyl, and methoxy, and m is selected from 0 or 1.

In a preferred embodiment of the present disclosure, the compound has a structure selected from: 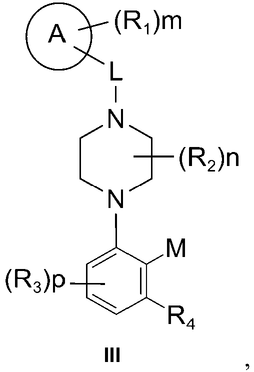 where ring A, R₁, m, R₂, n, R₃, p, M, L, and R₄ are as defined in the first aspect of the present disclosure.

In a preferred embodiment of the present disclosure, the compound has a structure selected from: 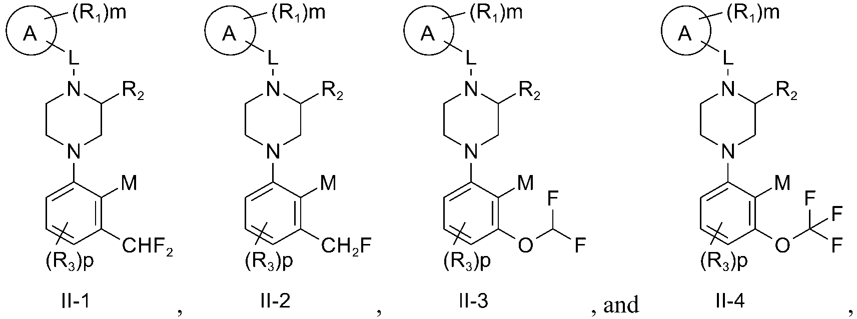 where ring A, M, L, R₁, R₂, R₃, m, and p are as defined in the first aspect of the present disclosure.

In a preferred embodiment of the present disclosure, the compound has a structure selected from: 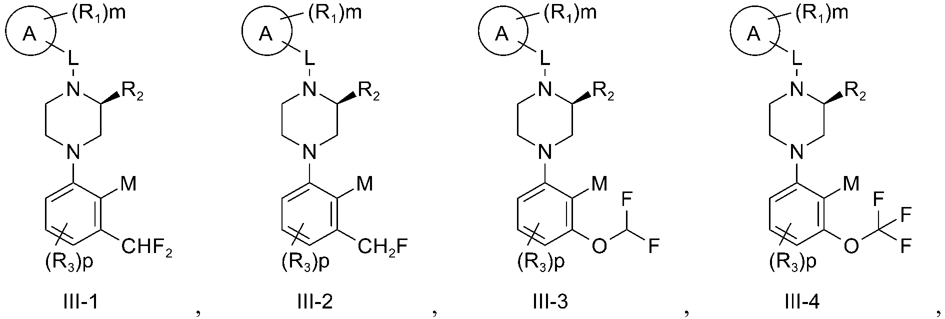 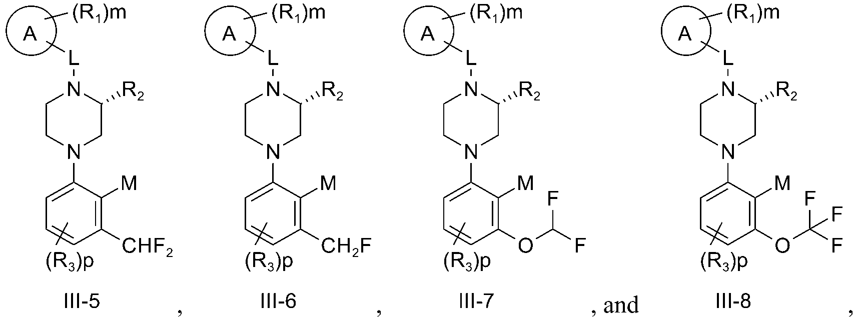 where ring A, M, L, R₁, R₂, R₃, m, and p are as defined in the first aspect of the present disclosure.

In a preferred embodiment of the present disclosure, the compound has a structure selected from: 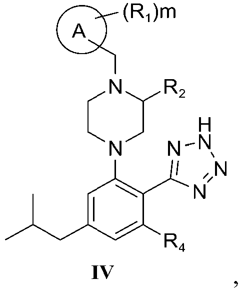 where ring A, R₁, m, R₂, and R₄ are as defined in the first aspect of the present disclosure.

In a preferred embodiment of the present disclosure, the compound represented by Formula IV has a structure represented by Formula IVa or Formula IVb: 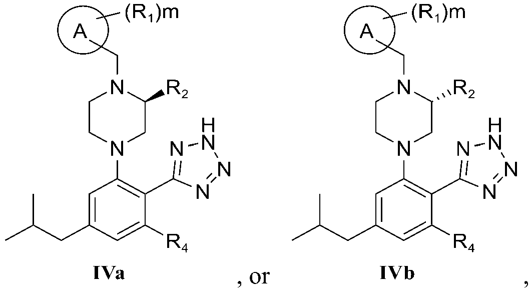 where ring A, R₁, m, R₂, and R₄ are as defined in the first aspect of the present disclosure.

In a preferred embodiment of the present disclosure, the compound has a structure selected from: 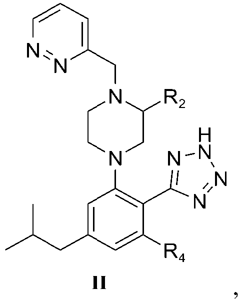 where R₂ and R₄ are as defined in the first aspect of the present disclosure.

In a preferred embodiment of the present disclosure, the compound has a structure represented by Formula IIa or Formula IIb: 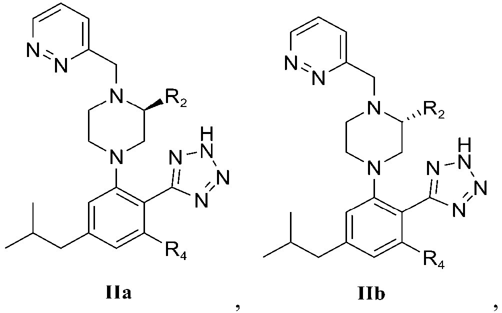 where R₂ and R₄ are as defined in the first aspect of the present disclosure.

In a preferred embodiment of the present disclosure, the compound has a structure selected from: 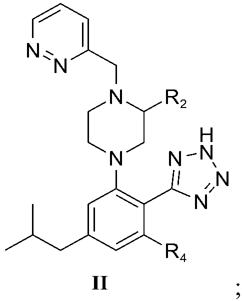 and
preferably, the compound has a structure represented by Formula IIa or Formula IIb:

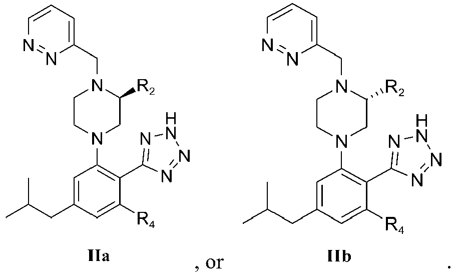

In a preferred embodiment of the present disclosure, the compound has a structure selected from: 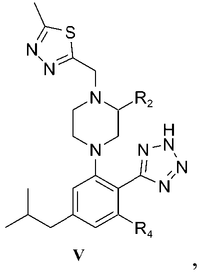 where R₂ and R₄ are as defined in the first aspect of the present disclosure.

In a preferred embodiment of the present disclosure, the compound represented by Formula V has a structure represented by Formula Va or Formula Vb: 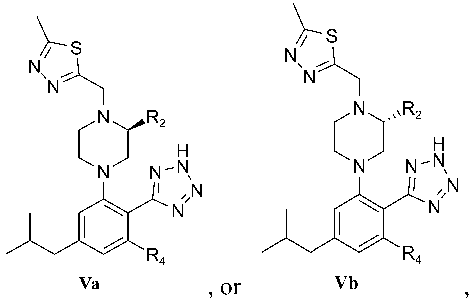 where R₂ and R₄ are as defined in the first aspect of the present disclosure.

In a preferred embodiment of the present disclosure, R₂ is selected from oxo (=O), C₁ to C₃ alkyl, C₁ to C₃ alkoxy, C₃ to C₆ cycloalkyl, a 5- to 6-membered heterocyclic ring, and a benzene ring, wherein the C₁ to C₃ alkyl, the C₁ to C₃ alkoxy, the C₃ to C₆ cycloalkyl, the 5-to 6-membered heterocyclic ring, and the benzene ring are optionally substituted with one, two, three, or four substituents selected from halogen, hydroxyl, cyano, amino, C₁ to C₃ alkyl, C₁ to C₃ alkoxy, and C₃ to C₆ cycloalkyl.

In a preferred embodiment of the present disclosure, R₂ is selected from oxo (=O), C₁ to C₃ alkyl, and halogenated C₁ to C₄ alkyl, and n is selected from 0, 1, 2, 3, or 4.

In a preferred embodiment of the present disclosure, R₂ is selected from methyl and halogenated methyl, and n is selected from 0 or 1.

In a preferred embodiment of the present disclosure, R₂ is selected from methyl and halogenated methyl, and n is selected from 1.

In a preferred embodiment of the present disclosure, R₂ is selected from methyl, and n is selected from 1.

In a preferred embodiment of the present disclosure, R₃ is selected from halogen, C₁ to C₆ alkyl, C₁ to C₆ alkoxy, and C₃ to C₇ cycloalkyl, wherein the C₁ to C₆ alkyl, the C₁ to C₆ alkoxy, and the C₃ to C₇ cycloalkyl are optionally substituted with one to four substituents selected from halogen, hydroxyl, cyano, C₁ to C₆ alkyl, C₁ to C₆ alkoxy, and C₃ to C₇ cycloalkyl, and p is selected from 0, 1, 2, 3, or 4.

In a preferred embodiment of the present disclosure, R₃ is selected from F, C₁ to C₅ alkyl, halogenated C₁ to C₅ alkyl, and methyl-cyclopropyl, and p is selected from 1, 2, and 3.

In a preferred embodiment of the present disclosure, R₃ is selected from F, methyl, trifluoromethyl, isobutyl, fluorinated isobutyl, and methyl-cyclopropyl, and p is selected from 1.

In a preferred embodiment of the present disclosure, R₃ is selected from isobutyl, and p is selected from 1.

In a preferred embodiment of the present disclosure, R₄ is selected from -CHF₂, - CH₂F, and -(Co to C₃ alkyl)-O-(halogenated C₁ to C₃ alkyl), wherein the -(Co to C₃ alkyl)-O-(halogenated C₁ to C₃ alkyl), if present, is optionally substituted with one to four substituents selected from hydroxyl, amino, C₃ to C₇ heterocycloalkyl, C₁ to C₃ alkyl, C₃ to C₆ cycloalkyl, and C₁ to C₃ alkoxy.

In a preferred embodiment of the present disclosure, R₄ is selected from -CHF₂, - CH₂F, and -O-(halogenated C₁ to C₃ alkyl).

In a preferred embodiment of the present disclosure, R₄ is selected from -CHF₂, - CH₂F, and -O-(C₁ to C₃ fluoroalkyl).

In a preferred embodiment of the present disclosure, R₄ is selected from -CHF₂, - CH₂F, and -O-fluoromethyl.

In a preferred embodiment of the present disclosure, R₄ is selected from -CHF₂ and -O-(C₁ to C₃ fluoroalkyl).

In a preferred embodiment of the present disclosure, R₄ is selected from -CHF₂, - CH₂F, -O-CHF₂, and -O-CF₃.

In a preferred embodiment of the present disclosure, M is selected from carboxylic group, -CONHSO₂R₅, -SO₂NHCOR₅, tetrazole, or a tetrazole bioisostere.

In a preferred embodiment of the present disclosure, M is selected from carboxylic group, -CONHSO₂R₅, -SO₂NHCOR₅, and tetrazole, wherein R₅ is selected from C₁ to C₃ alkyl and C₃ to C₅ cycloalkyl.

In a preferred embodiment of the present disclosure, M is selected from tetrazole.

In a preferred embodiment of the present disclosure, L is absent or selected from methylene, -CH₂CH₂-, -CH(CH₃)-, -CH₂CH₂CH₂-, -CH₂CH(CH₃)-, -O-CH₂-, and -NH-CH₂-.

In a preferred embodiment of the present disclosure, L is selected from methylene, -CH₂CH₂-, -CH(CH₃)-, and -O-CH₂-.

In a preferred embodiment of the present disclosure, L is selected from methylene.

In a preferred embodiment of the present disclosure, the compound has a structure selected from: 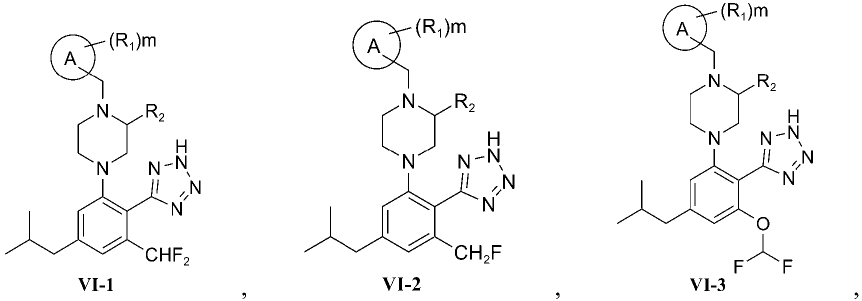 and 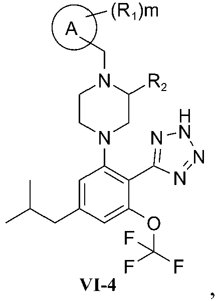 where ring A, R₁, R₂, and m are as defined in the first aspect of the present disclosure.

In a preferred embodiment of the present disclosure, the compound represented by Formula VI-1 has a structure represented by Formula VI-1a or Formula VI-1b: 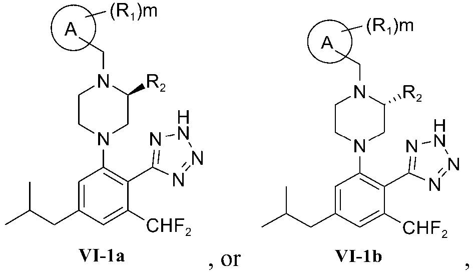 where ring A, R₁, R₂, and m are as defined in the first aspect of the present disclosure.

In a preferred embodiment of the present disclosure, the compound represented by Formula VI-2 has a structure represented by Formula VI-2a or Formula VI-2b: 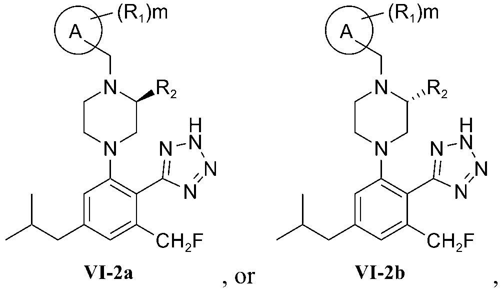 where ring A, R₁, R₂, and m are as defined in the first aspect of the present disclosure.

In a preferred embodiment of the present disclosure, the compound represented by Formula VI-3 has a structure represented by Formula VI-3a or Formula VI-3b: 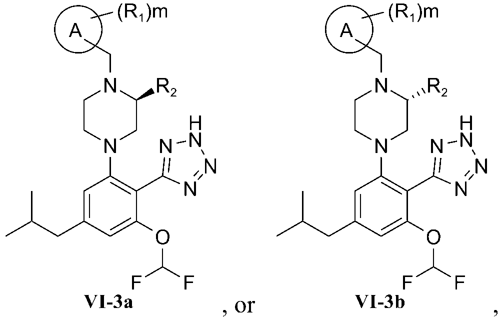 where ring A, R₁, R₂, and m are as defined in the first aspect of the present disclosure.

In a preferred embodiment of the present disclosure, the compound represented by Formula VI-4 has a structure represented by Formula VI-4a or Formula VI-4b: 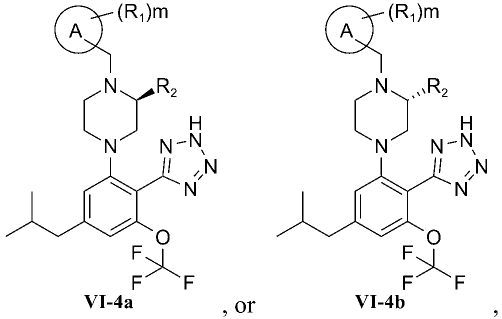 where ring A, R₁, R₂, and m are as defined in the first aspect of the present disclosure.

In a preferred embodiment of the present disclosure, 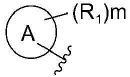 is 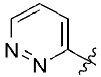 or

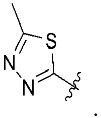

In a preferred embodiment of the present disclosure, 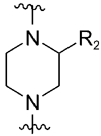 is

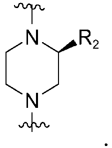

In a preferred embodiment of the present disclosure, for the compound represented by Formula I, or the tautomer, stereoisomer, or pharmaceutically acceptable salt thereof, the compound includes:

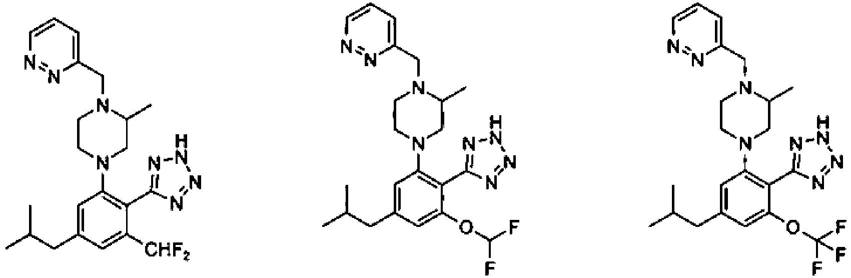

In a preferred embodiment of the present disclosure, for the compound represented by Formula I, or the tautomer, stereoisomer, or pharmaceutically acceptable salt thereof, the compound includes:

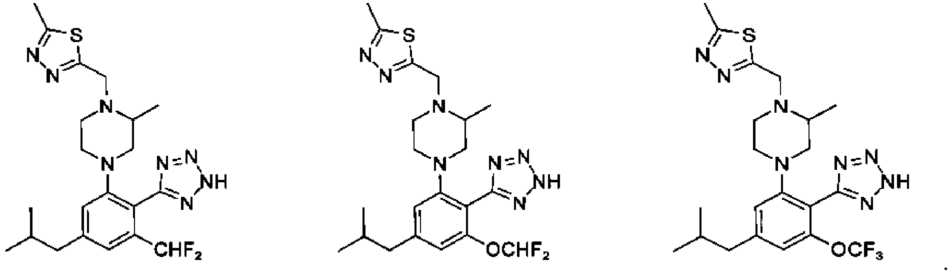

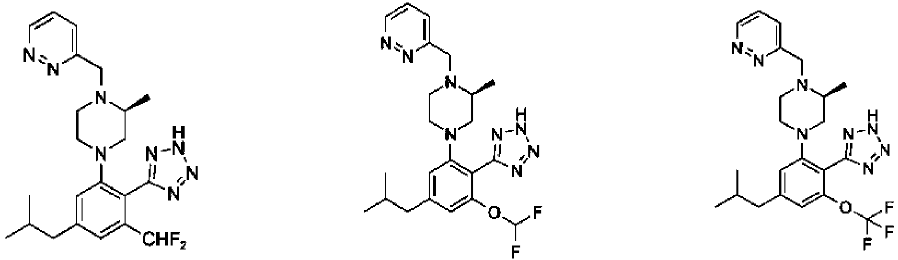

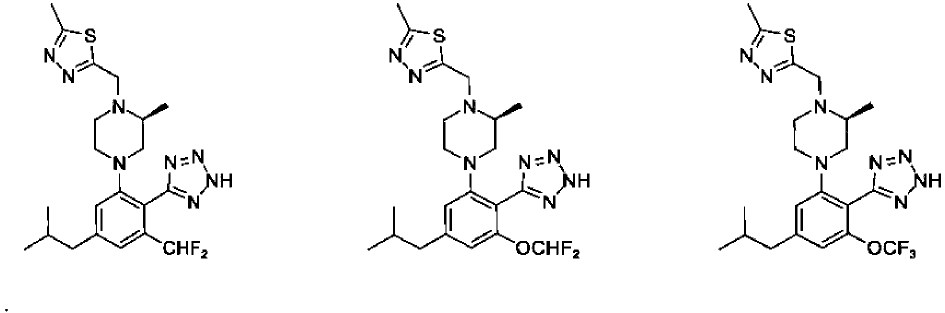

In a second aspect of the present disclosure, there is provided a pharmaceutical composition. The pharmaceutical composition includes the compound represented by Formula I, or the tautomer, stereoisomer, or pharmaceutically acceptable salt thereof according to the first aspect of the present disclosure and a pharmaceutically acceptable carrier.

In a third aspect of the present disclosure, there is provided use of the compound represented by Formula I, or the tautomer, stereoisomer, or pharmaceutically acceptable salt thereof according to the first aspect of the present disclosure, or the pharmaceutical composition according to the second aspect of the present disclosure. The use includes:
acting as an AT2R antagonist; and/or
preventing and/or treating an AT2R-mediated disease; and/or
preventing and/or treating a disease where a reduced effect of Ang II is desired or required; and/or
preparing a medicament, pharmaceutical composition, or formulation as an AT2R antagonist; and/or
preparing a medicament, pharmaceutical composition, or formulation for preventing and/or treating a disease where AT2R is expressed and an inhibition of AT2R is desired or necessary.

Provided is the compound represented by Formula I, or the tautomer, stereoisomer, or pharmaceutically acceptable salt thereof according to the first aspect of the present disclosure, or the pharmaceutical composition according to the second aspect of the present disclosure for prevention or treatment of a disease.

Provided is the compound represented by Formula I, or the tautomer, stereoisomer, or pharmaceutically acceptable salt thereof according to the first aspect of the present disclosure, or the pharmaceutical composition according to the second aspect of the present disclosure for treatment of neuropathy or neuropathic pain.

The neuropathy to be mentioned includes primary neuropathy, secondary neuropathy, peripheral neuropathy, neuropathy caused by mechanical nerve injury or biochemical nerve injury, or a diabetes-related neuropathic disease.

The neuropathic pain to be mentioned includes postherpetic neuralgia or diabetic neuropathic pain.

The compound represented by Formula I, or the tautomer, stereoisomer, or pharmaceutically acceptable salt thereof according to the first aspect of the present disclosure, or the pharmaceutical composition according to the second aspect of the present disclosure is suitable for the treatment and/or preventive treatment of the aforementioned diseases.

A fourth aspect of the present disclosure provides a method for treating a disease. The disease is: an AT2R-mediated disease; and/or a disease where a reduced effect of Ang II is desired or necessary; and/or a disease where AT2R is expressed and an inhibition of AT2R is desired or necessary. The method includes: administering a therapeutically effective dose of the compound represented by Formula I, or the tautomer, stereoisomer, or pharmaceutically acceptable salt thereof according to the first aspect of the present disclosure, or the pharmaceutical composition according to the second aspect of the present disclosure to a subject suffering from or susceptible to the disease.

Additional aspects and advantages of the embodiments of the present disclosure will be provided at least in part in the following description, or will become apparent in part from the following description, or can be learned from the practice of the embodiments of the present disclosure.

### Terms and Definitions

Unless otherwise stated, the definitions of groups and terms described in the specification and claims include actual definitions, exemplary definitions, preferred definitions, definitions recorded in tables, and definitions of specific compounds in the examples, etc., which may be arbitrarily combined and integrated with each other. The group definitions and compound structures that are combined and integrated should fall within the scope of the present disclosure.

Unless defined otherwise, all technical and scientific terms herein have the same meaning as commonly understood by one of ordinary skill in the art to which the claimed subject matter belongs. The patents, patent applications, publications cited herein are hereby incorporated by reference in their entireties, unless stated otherwise. When a term has multiple definitions, the definition in this section will prevail.

It should be understood that the foregoing general description and the following detailed description are illustrative and merely for explanation, rather than limiting the subject matter of the present disclosure in any way. In the present disclosure, unless specifically stated otherwise, the use of the singular also includes the plural. It must be noted that, as used in this specification and the claims, the singular forms include plural referents unless the context clearly dictates otherwise. It should also be noted that the use of "or" or "either" means "and/or" unless stated otherwise. Furthermore, use of the term "include", as well as other forms, such as "comprising", "including", and "containing", is not for limitation.

Definitions of standard chemical terms may be found in reference document (including "ADVANCED ORGANIC CHEMISTRY 4THED.", Carey and Sundberg, Vols. A(2000)and B(2001), Plenum Press, New York). Unless otherwise indicated, conventional methods in the related art, for example, mass spectroscopy, NMR, IR and UV/VIS spectroscopy, and pharmacological methods, are employed. Unless specific definitions are set forth, the terms in the related description of analytical chemistry, organic synthetic chemistry, and medicinal and medicinal chemistry are those known in the art. Standard techniques may be used in chemical synthesis, chemical analysis, pharmaceutical preparation, formulation and delivery, and treatment of patients. For example, reactions and purifications can be performed using the manufacturer's instructions of the kit, or in a manner well known in the related art or as described herein. The techniques and procedures described above may generally be performed with conventional methods well known in the art according to the description in a number of general and more specific documents cited and discussed throughout the present description. Throughout the description, groups and substituents thereof can be chosen by one skilled in the field to provide stable moieties and compounds.

Where substituent groups are depicted by conventional chemical formulae, written from left to right, the substituent groups also encompass the chemically equivalent substituents that would result from writing the structural formula from right to left. For example, CH₂O is equivalent to OCH₂. As used herein, 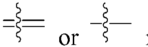 refers to the point of attachment of a group. As used herein, "R₁", "R1", and "R¹" have the same meaning and are interchangeable. For other symbols such as R₂, similar definitions have the same meanings.

The section headings used herein are for organizational purposes only and are not to be construed as limiting the subject matter described. All documents or portions of documents cited in the present disclosure, including but not limited to patents, patent applications, articles, books, manuals, and treatises, are incorporated herein by reference in their entireties.

In addition to the foregoing, the following terms, when used in the specification and claims of the present disclosure, have the meanings indicated below, unless otherwise specifically stated.

As used herein, numerical ranges recited in the specification and claims, when interpreted as "integers", should be understood as including both endpoints of the range and each integer within the range. For example, "an integer ranging from 1 to 6" should be understood as reciting each integer of 1, 2, 3, 4, 5, and 6.

In the present disclosure, when the number of substituents is specified, the term "one or more" refers to a range of the number of substitution from one substituent up to the maximum possible number of substituents, i.e., from replacement of one hydrogen atom to replacement of all hydrogen atoms by substituents. When the number of substituents is specified, the term "1 to 4" refers to 1, 2, 3, or 4 substitutions, i.e., substitution of 1, 2, 3, or 4 hydrogen atoms by the substituents.

In the present disclosure, the term "saturated, partially saturated, or unsaturated" includes substituents that are saturated with hydrogen, substituents that are completely unsaturated with hydrogen, and substituents that are partially saturated with hydrogen.

In the present disclosure, "AT2 receptor" and "AT2R" have the same definition.

In the present disclosure, the term "ortho" refers to a relationship between two moieties bonded to adjacent atoms. For example, in a residue -CHRₓ-CHR_{y}-, Rₓ and R_{y} are ortho groups, and Rₓ may be referred to as an ortho R group of Ry, including but not limited to

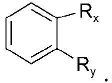

In the present disclosure, the term "bioisostere", either alone or as part of other substituents, has its ordinary meaning in the art and refers to a group or substituent having similar physical or chemical properties and having similar biological activity to another said group or substituent. The bioisostere is typically obtained by replacing one atom or group with another substantially similar atom or group and may have one or more enhanced properties compared to the original compound, group, or substituent (such as reduced toxicity, increased bioavailability, altered or enhanced activity, or improved metabolism). The term "tetrazole bioisostere" refers to a substance that has similar physical and chemical properties to tetrazole in a biological context and exhibits similar or related biological activity as a result of such properties. In some embodiments, the term "tetrazole bioisostere" includes, but is not limited to, -C(=O)-R₅, -C(=O)NH-R₅, -N(R₅) C(=O)-R₅, -O-N(R^{e1})C(=O)-R₅, -S(=O)-R₅, -S(=O)₂-R₅, -NH-S(=O)₂-R₅, -C(=O)-NH-S(=O)₂-R₅, -NH-C(=O)-NH-S(=O)₂-R₅, -NH-C(=O)-NH-C(=O)-R₅, -P(=O)(-R₅)₂, and 4- to 8-membered heterocycloalkyl substituted with one, two, three, or four oxo (=O), wherein R₅ is selected from H, OH, NH₂, ONa, OK (potassium ion), C₁ to C₈ alkyl, C₃ to C₈ cycloalkyl, C₁ to C₈ alkoxy, and a 5- to 6-membered heterocyclic ring, wherein the C₁ to C₈ alkyl, the C₃ to C₈ cycloalkyl, the C₁ to C₈ alkoxy and the 5- to 6-membered heterocyclic ring are optionally substituted with one to four substituents selected from halogen, amino, C₃ to C₇ heterocycloalkyl, C₁ to C₃ alkyl, C₃ to C₆ cycloalkyl, and C₁ to C₃ alkoxy. The 4- to 8-membered heterocycloalkyl is as defined by the term "heterocycloalkyl" below. For example, the term "tetrazolium bioisostere" includes, but is not limited to, phosphoric acid,

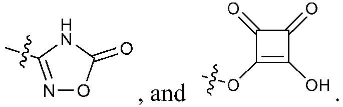

In the present disclosure, the term "halogen" refers to fluorine, chlorine, bromine, and iodine, either alone or as part of other substituents.

In the present disclosure, the term "amino" refers to -NH₂, either alone or as part of other substituents.

In the present disclosure, the term "hydroxy" refers to -OH, either alone or as part of other substituents.

In the present disclosure, the term "cyano" refers to -CN, either alone or as part of other substituents.

In the present disclosure, the term "alkyl", either alone or as part of other substituents, means a straight or branched hydrocarbon chain group consisting solely of carbon and hydrogen atoms, containing no unsaturated bonds, and having, for example, 1 to 6 carbon atoms and connected to the rest of the molecule by a single bond. Examples of alkyl include, but are not limited to, methyl, ethyl, n-propyl, isopropyl, n-butyl, isobutyl, tert-butyl, pentyl, isopentyl, neopentyl, and hexyl. Alkyl may be unsubstituted or substituted with one or more suitable substituents. Alkyl may also be an isotopic isomer of the naturally abundant alkyl enriched in an isotope of carbon and/or hydrogen (i.e., deuterium or tritium). As used herein, the term "alkenyl" refers to an unbranched or branched monovalent hydrocarbon chain containing one or more carbon-carbon double bonds. As used herein, the term "alkynyl" refers to an unbranched or branched monovalent hydrocarbon chain containing one or more carbon-carbon triple bonds.

In the present disclosure, the term "C₁ to C₆ alkyl", either alone or as part of other substituents, should be interpreted as a straight or branched chain saturated hydrocarbon group having 1, 2, 3, 4, 5, or 6 carbon atoms. Examples of "C₁ to C₆ alkyl" are, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, or isomers thereof. In particular, the group has 1, 2, or 3 carbon atoms ("C₁ to C₃ alkyl"), for example methyl, methylene, ethyl, n-propyl, or isopropyl.

In the present disclosure, unless otherwise specified, the term "alkylene" should be interpreted as a straight divalent hydrocarbon group having 1 to 6 carbon atoms or a branched divalent hydrocarbon group having 3 to 6 carbon atoms, such as methylene, ethylene, propylene, 1-methylpropylene, and butylene.

In the present disclosure, the term "C₁ to C₆ alkoxy", either alone or as part of other substituents, should be interpreted to include a straight or branched saturated hydrocarbon group having 1, 2, 3, 4, 5, or 6 carbon atoms and an oxygen atom, or present as C₁ to C₆ alkyl-O-C₁ to C₆ alkyl as defined in the present specification, the oxygen atom being attached to any carbon atom of the straight or branched chain of the C₁ to C₆ alkyl, including but not limited to: methoxy (CH₃-O-), ethoxy (C₂H₅-O-), propoxy (C₃H₇-O-), and butoxy (C₄H₉-O-).

In the present disclosure, "halogenated alkoxy", either alone or as part of other substituents, refers to an alkoxy as described above, in which any number (at least one) of hydrogen atoms attached to alkoxy is substituted with fluorine, chlorine, bromine, or iodine.

In the present disclosure, the term "oxo", either alone or as part of other substituents, refers to that two hydrogen atoms of methylene group are substituted with oxygen atoms, i.e., the methylene group is substituted with a carbonyl group, denoted as =O.

In the present disclosure, "halogenated alkyl", either alone or as part of other substituents, refers to a saturated aliphatic hydrocarbon group including branched and straight chains having a specified number of carbon atoms, and substituted with one or more halogens (e.g., -CᵥF_{w}, where v=1 to 3, and w=1 to (2v+1)). Examples of halogenated alkyl include, but are not limited to, trifluoromethyl, trichloromethyl, pentafluoroethyl, pentachloroethyl, 2,2,2-trifluoroethyl, heptafluoropropyl, and heptachloropropyl.

In the present disclosure, the term "aryl" or "aromatic ring", either alone or as part of other substituents, refers to a monocyclic or polycyclic carbocyclic ring having 6 to 20 carbon atoms, in which at least one ring is aromatic. When one of the rings is a non-aromatic ring, the group may be attached via the aromatic ring or the non-aromatic ring. Examples of aryl include, but are not limited to, phenyl, naphthyl, tetrahydronaphthyl, 2,3-dihydroindenyl, biphenyl, phenanthrenyl, anthracenyl, and acenaphthenyl.

In the present disclosure, the term "heterocyclic ring", either alone or as part of other substituents, includes "heteroaromatic ring", "heterocycloalkenyl", and "heterocycloalkyl", and refers to a cycloalkyl, cycloalkenyl, or aromatic ring group in which one or more (in some embodiments, 1 to 3) carbon atoms are replaced with heteroatoms such as, but not limited to, N, O, S, and P.

In the present disclosure, the term "heteroaromatic ring", either alone or as part of other substituents, refers to a monocyclic or polycyclic carbocyclic ring, in which at least one ring atom is a heteroatom independently selected from oxygen, sulfur, and nitrogen, and the remaining ring atoms are C, and in which at least one ring is aromatic. The group may be a carbon group or a heteroatom group (i.e., C-attached or N-attached, as long as this is possible). When one of the rings is a non-aromatic ring, the group may be attached via the aromatic ring or the non-aromatic ring. Examples of heteroaryl include, but are not limited to, imidazolyl, acridinyl, carbazolyl, cinnolinyl, quinoxalinyl, pyrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl, pyrrolyl, N-methylpyrrolyl, and tetrahydroquinoline. The term "heteroaromatic ring" may be used interchangeably with the term "heteroaromatic ring system", "heteroaryl" or "heteroaromatic ring group".

In the present disclosure, the term "5- to 10-membered heteroaromatic ring", either alone or as part of other substituents, may be used interchangeably with "5- to 10-membered heteroaryl", and should be interpreted as an aromatic ring group having 5 to 10 ring atoms and containing 1 to 5 heteroatoms independently selected from N, O, and S. The term "5- to 6-membered heteroaromatic ring" should be interpreted as an aromatic ring group having 5 or 6 ring atoms and containing 1 to 3 heteroatoms independently selected from N, O, and S. In particular, heteroaryl is selected from thienyl, furanyl, pyrrolyl, oxazolyl, thiazolyl, imidazolyl, pyrazolyl, pyridinyl, pyridazinyl, pyrimidinyl, and pyrazinyl.

In the present disclosure, the term "heterocycloalkenyl", either alone or as part of other substituents, refers to a monocyclic group containing heteroatoms (the monocyclic group has double bonds but no aromaticity), and preferably, a monocyclic ring containing 1, 2, or 3 ring heteroatoms independently selected from N, O and S. Examples of heterocycloalkenyl includes: dihydrofuryl, dihydrothienyl, dihydropyrrolyl, dioxolenyl, dihydroimidazolyl, dihydropyrazolyl, dihydrothiazolyl, dihydroisothiazolyl, dihydroxadiazolyl, dihydrothiazolyl, dihydrotriazolyl, dihydrotetrazolyl, tetrahydropyridinyl, 3,4-dihydro-2H-pyran, pyranyl, thiopyranyl, dihydropyridinyl, dihydropyrazinyl, dihydropyrimidinyl, oxazinyl, dihydrotetrazolyl, and the like. The term "heteroalkene ring" may be used interchangeably with the term "heterocycloalkenyl".

In the present disclosure, the term "heterocycloalkyl", either alone or as part of other substituents, refers to cycloalkyl in which one or more (in some embodiments, 1 to 3) carbon atoms are replaced with heteroatoms such as, but not limited to, N, O, S, and P. "Heterocycloalkyl" may be saturated or unsaturated, but not aromatic. "Heterocycloalkyl" may also contain 1, 2, or 3 rings, including bridged rings and spiro ring structures. The term "5- to 10-membered heterocyclyl" or "5- to 10-membered heterocycloalkyl" should be interpreted as a monocyclic, bicyclic or tricyclic ring having 5 to 10 atoms, in which the heteroatoms are preferably selected from N, O and S. It should be understood that when the total number of S and O atoms in heterocyclyl exceeds one, these heteroatoms are not adjacent to each other. Examples of heterocycloalkyl include, but are not limited to, tetrahydroisoquinolinyl, tetrahydroquinolinyl, tetrahydropyranyl, tetrahydrofuranyl, and tetrahydrothiopyranyl.

In the present disclosure, the term "cycloalkyl" or "carbocyclyl", either alone or as part of other substituents, refers to a cyclic alkyl. The term "m- to n-membered cycloalkyl" or "Cₘ to Cₙ cycloalkyl" should be interpreted as a saturated, unsaturated, or partially saturated carbocyclic ring having m to n atoms. For example, "3- to 15-membered cycloalkyl" or "C₃ to C₁₅ cycloalkyl" refers to a cyclic alkyl group containing 3 to 15, 3 to 9, 3 to 6, or 3 to 5 carbon atoms, which may contain 1 to 4 rings. "5- to 8-membered cycloalkyl" contains 5 to 8 carbon atoms. Examples of unsubstituted cycloalkyl include, but are not limited to, cyclopropyl, cyclobutyl, cyclopentyl, cyclohexyl, and adamantyl, or a bicyclic hydrocarbon group such as a decalin ring. Cycloalkyl may be substituted with one or more substituents. The term "C₃ to C₇ cycloalkyl" should be interpreted as a saturated monocyclic or bicyclic hydrocarbon ring having 3 to 7 carbon atoms, for example, cyclopropyl, cyclobutyl, cyclopentyl, and cyclohexyl.

In the present disclosure, "halogenated cycloalkyl", either alone or as part of other substituents, refers to a cycloalkyl as described above, in which any number (at least one) of hydrogen atoms attached to cycloalkyl are substituted with fluorine, chlorine, bromine, or iodine.

In the present disclosure, the term "monocyclic ring", either alone or as part of other substituents, refers to a group with only one ring, which may be saturated, unsaturated, or partially saturated, and may be a carbocyclic ring (all ring atoms are carbon atoms) or a heterocyclic ring (in addition to carbon atoms, ring atoms include, for example, 1, 2, or 3 heteroatoms, such as N, O, or S).

The compound according to the present disclosure includes an intermediate useful for preparing the compound according to the present disclosure, which contains reactive functional groups (such as, but not limited to, carboxyl, hydroxyl, and amino moieties), and also includes protected derivatives thereof. "Protected derivatives" are those compounds in which one or more reactive sites are blocked by one or more protecting groups (also referred to as protective groups). Suitable protecting groups for carboxyl moieties include benzyl, tert-butyl, and the like, as well as isotopes and the like. Suitable protecting groups for amino and amido include acetyl, trifluoroacetyl, tert-butoxycarbonyl, benzyloxycarbonyl, and the like. Suitable protecting groups for hydroxyl moieties include benzyl and the like. Other suitable protecting groups are well-known to those skilled in the art.

In the present disclosure, the term "substituted" means that any one or more hydrogen atoms on a specified atom are replaced with one or more substituents, including deuterium and hydrogen variants, as long as the valence of the specified atom is normal and the substituted compound is stable.

In the present disclosure, the term "optional" or "optionally" means that the subsequently described event or situation may or may not occur, and such an expression includes both occurrences and non-occurrences of the event or condition. For example, "optionally substituted aryl" means that the aryl is substituted or unsubstituted, and such an expression includes both substituted and unsubstituted aryl.

In the present disclosure, the term "optionally replaced with..." or "optionally substituted with..." means that a specified group is unsubstituted or substituted with one or more substituents independently selected from possible substituents. For example, "aryl is optionally substituted with one to four substituents independently selected from halogen, cyano, hydroxyl, and C₁₋₆ alkyl" means that aryl is unsubstituted or substituted with one, two, three, or four substituents independently selected from halogen, cyano, hydroxyl, and C₁₋₆ alkyl, and the description encompasses both substituted aryl and unsubstituted aryl.

In the present disclosure, the term "salt" or "pharmaceutically acceptable salt" includes pharmaceutically acceptable acid addition salts and pharmaceutically acceptable base addition salts. The term "pharmaceutically acceptable" means that the compounds, materials, compositions, and/or dosage forms are suitable for contact with human and animal tissues without excess toxicity, irritation, allergic reactions, or other problems or complications within the scope of reliable medical judgment, and are commensurate with a reasonable benefit/risk ratio.

In the present disclosure, the term "pharmaceutically acceptable acid addition salt" refers to a salt formed with an inorganic acid or organic acid that retains the biological effectiveness of the free base without other adverse effects. "Pharmaceutically acceptable base addition salt" refers to a salt formed with an inorganic base or an organic base that retains the biological effectiveness of the free acid without other adverse effects. In addition to the pharmaceutically acceptable salts, other salts may be adopted in the present disclosure, and they can serve as intermediates in the purification of compounds or in the preparation of other pharmaceutically acceptable salts, or can be used for identifying, characterizing, or purifying the compounds of the present disclosure.

In the present disclosure, the term "amine salt" refers to a product obtained by neutralizing an alkyl primary, secondary, or tertiary amine with an acid. The acid includes the inorganic acid or organic acid described in the present disclosure.

In the present disclosure, the term "stereoisomer" refers to isomers formed due to different spatial arrangements of atoms in a molecule, including a cis-trans isomer, an enantiomer, a diastereoisomer, and a conformational isomer.

According to selected raw materials and methods, the compound of the present disclosure may be present in the form of a possible isomer or a mixture of isomers, for example, in the form of a pure optical isomer or a mixture of isomers such as a mixture of racemate and diastereoisomer, depending on the number of asymmetric carbon atoms. When an optically active compound is described, prefixes *D* and *L,* or *R* and *S* are used to represent an absolute configuration of a molecule with respect to a chiral center (or multiple chiral centers) in the molecule. Prefixes *D* and *L,* or (+) and (-) are symbols used for designating the rotation of plane polarized light caused by a compound, and (-) or *L* indicates that a compound is levorotatory. Compounds with the prefix (+) or *D* are dextrorotatory.

When a bond to a chiral carbon in the formula of the present disclosure is depicted as a straight line, it is to be understood that (*R*) and (*S*) configurations of the chiral carbon and produced enantiomerically pure compounds and mixtures are all included within the scope of the general formula. The racemate or enantiomerically pure compounds of the present disclosure are graphically represented with reference to Maehr, J. Chem. Ed. 1985, 62: 114-120. A wedge bond and a dashed bond are used to represent an absolute configuration of a stereocenter.

In the present disclosure, the term "tautomer" refers to functional group isomers formed by rapid movement of a certain atom in a molecule between two positions. The compound of the present disclosure may have a tautomerism phenomenon. The tautomeric compound may be present in two or more interconvertible forms. A prototropic tautomer is formed by migration of a hydrogen atom covalently bonded between two atoms. Tautomer is generally present in an equilibrium form, and separation of a single tautomer usually yields a mixture whose physicochemical properties are consistent with those of a mixture of compounds. The position of equilibrium depends on intramolecular chemical properties. For example, in several aliphatic aldehydes and ketones such as acetaldehyde, ketonic configurations prevail, while in phenol, an enolic configuration prevails. The present disclosure covers all tautomer of the compound.

In the present disclosure, "pharmaceutical composition" refers to a formulation of the compound of the present disclosure and a medium commonly accepted in the art for delivery of a biologically active compound to a mammal (e.g., a human). The medium includes a pharmaceutically acceptable carrier. The purpose of the pharmaceutical composition is to facilitate administration to an organism, facilitate the absorption of the active ingredients, and further facilitate the exertion of the biological activity.

In the present disclosure, "pharmaceutically acceptable carrier" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier approved by the relevant governmental regulatory authorities as acceptable for human or livestock use.

The compound of the present disclosure may contain an unnatural proportion of atomic isotopes at one or more atoms forming the compound. For example, the compound can be labeled with radioisotopes such as deuterium (²H), tritium (³H), iodine-125 (¹²⁵I), and C-14 (¹⁴C). All changes made to the isotope composition of the compound of the present disclosure, regardless of whether they are radioactive or not, shall fall within the scope of the present disclosure.

In the present disclosure, the term "adjuvant" refers to medicinal inert ingredients. Examples of types of the term "excipient" include, but are not limited to, an adhesive, a disintegrant, a lubricant, a glidant, a stabilizer, a filler, a diluent, etc. Excipients can enhance the operating characteristics of pharmaceutical preparations, that is, improve the fluidity and/or adhesiveness to enable preparations to be more suitable for direct compression.

As used herein, the term "treatment" and other similar synonyms include the following meanings: (i) preventing a disease or condition from occurring in a mammal, particularly where such mammal is susceptible to the disease or condition but has not yet been diagnosed as having the disease or condition; (ii) inhibiting a disease or condition, i.e., suppressing development thereof; (iii) alleviating a disease or condition, that is, enabling the state of the disease or condition to regress; or (iv) alleviating the symptoms caused by the disease or condition.

### Beneficial Effects

Through extensive and in-depth research, the inventors have unexpectedly developed a compound as an AT2R antagonist, and the compound has a structure represented in the present disclosure. The compound of the present disclosure can prevent or treat an AT2R-related disease or disorder, exhibits excellent pharmacokinetic properties, and possesses high safety and drug-like properties.

### DETAILED DESCRIPTION OF THE EMBODIMENTS

The present disclosure is further described below in conjunction with specific embodiments. It should be understood that the following description is only the most preferred embodiment of the present disclosure, and should not be regarded as limiting the protection scope of the present disclosure. On the basis of fully understanding the present disclosure, the experimental methods in the following embodiments for which specific conditions are not specified are generally carried out under conventional conditions or under conditions recommended by the manufacturer. Those skilled in the art can make non-essential changes to the technical solutions of the present disclosure, and such changes should fall within the protection scope of the present disclosure.

### Definitions

### Symbol or unit:

IC₅₀: half inhibitory concentration, which refers to the concentration at which half of the maximum inhibitory effect is achieved.
M: mol/L, for example, n-butyllithium (14.56 mL, 29.1 mmol, 2.5 M n-hexane solution) means a solution of n-butyllithium in n-hexane with a molar concentration of 2.5 mol/L.
N: equivalent concentration, for example, 2N hydrochloric acid means 2 mol/L hydrochloric acid solution.
RT: retention time

### Reagents:

DMF: N,N-dimethylformamide
DIPEA: N,N-diisopropylethylamine

### Test method:

LCMS: liquid chromatography-mass spectrometry
TLC: thin layer chromatography

### Intermediate A1: Preparation of Intermediate A1

### (S)-3-((2-methylpiperazin-1-yl)methyl)pyridazine

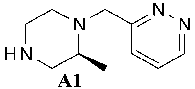

The synthetic scheme of Intermediate **A1** is as follows:

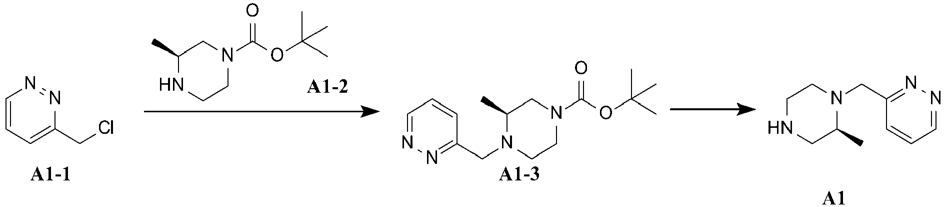

### Step 1: Synthesis of tert-butyl (S)-3-methyl-4-(pyridazin-3-ylmethyl)piperazine-1-carboxylate

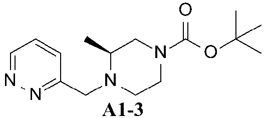

Triethylamine (2.4 g, 23.4 mmol) and tert-butyl (S)-3-methylpiperazine-1-carboxylate (1.56 g, 7.8 mmol) were added to a solution of 3-(chloromethyl)pyridazine (1 g, 7.8 mmol) in N,N-dimethylformamide (10 mL), and the mixture was then stirred at 50°C for 18 hours. The reaction solution was poured into water (100 mL) and extracted three times with 50 mL of ethyl acetate each time. The organic phase was dried over anhydrous sodium sulfate, filtered, and concentrated to obtain a crude product. The crude product was purified by column chromatography (petroleum ether/ethyl acetate (V/V)=1/0 to 1/1) to obtain the product tert-butyl (S)-3-methyl-4-(pyridazin-3-ylmethyl)piperazine-1-carboxylate (1.8 g, yield 79%).

LC-MS, M/Z (ESI): 293.4 [M+H]⁺

### Step 2: Synthesis of (S)-3-((2-methylpiperazin-1-yl)methyl)pyridazine

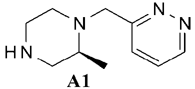

4M hydrochloric acid in 1,4-dioxane (9 mL) was added to a solution of tert-butyl (S)-3-methyl-4-(pyridazin-3-ylmethyl)piperazine-1-carboxylate (1.8 g, 6.2 mmol) in dioxane (9 mL), and the mixture reacted at room temperature for 2 hours. The reaction solution was concentrated to obtain the product (S)-3-((2-methylpiperazin-1-yl)methyl)pyridazine (1.5 g, hydrochloride).

LC-MS, M/Z (ESI): 193.3 [M+H]⁺

### Example 1: Preparation of Target Compound I-4

### (S)-3-((4-(3-(difluoromethyl)-5-isobutyl-2-(2H-tetrazol-5-yl)phenyl)-2-methylpiperazin-1-yl)methyl)pyridazine

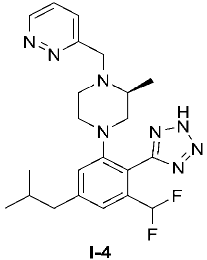

The synthetic scheme of Target Compound **I-4** is as follows:

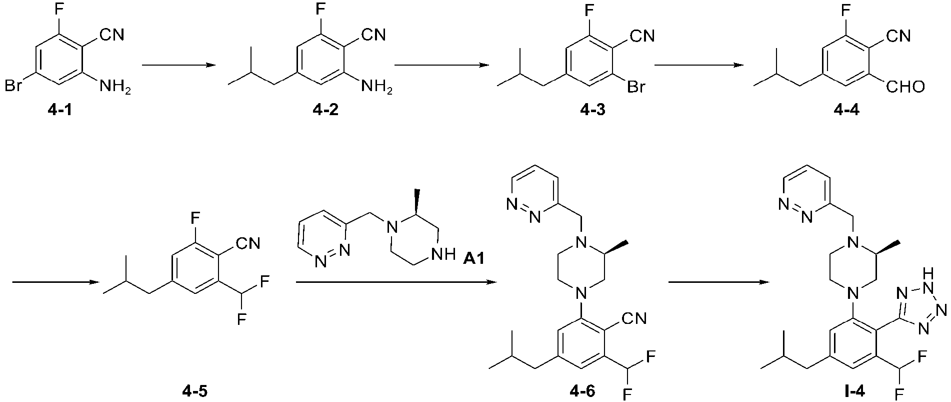

### Step 1: Synthesis of 2-amino-6-fluoro-4-isobutylbenzonitrile

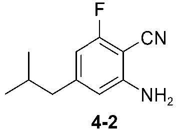

Under a nitrogen atmosphere, 1,4-dioxane (50 mL) was added to a mixture of 2-amino-4-bromo-6-fluorobenzonitrile (2.15 g, 10 mmol), isobutylboronic acid (3.06 g, 30 mmol), potassium carbonate (4.14 g, 30 mmol), and dichloro [1,1'-bis(diphenylphosphino) ferrocene] palladium (II) dichloromethane complex (362.5 mg, 0.5 mmol). Then, the mixture reacted at 80°C for 16 hours. After the reaction solution was cooled to room temperature, the reaction was quenched with 50 mL of water. The mixture was extracted three times with 50 mL of ethyl acetate each time. The organic phase was concentrated to dryness. The residue was adsorbed onto silica gel and purified by column chromatography (eluent: petroleum ether/ethyl acetate (V/V)=1:1) to obtain 2-amino-6-fluoro-4-isobutylbenzonitrile (1.74 g, yield: 90%).

LC-MS, M/Z (ESI): 193.1 [M+H]⁺

### Step 2: Synthesis of 2-bromo-6-fluoro-4-isobutylbenzonitrile

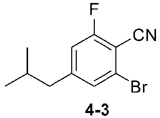

Under a nitrogen atmosphere, tert-butyl nitrite (2.78 g, 27 mmol) was added to a solution of 2-amino-6-fluoro-4-isobutylbenzonitrile (1.74 g, 9 mmol) and copper(I) bromide (3.87 g, 27 mmol) in anhydrous acetonitrile (45 mL). Then, the mixture reacted at room temperature for 4 hours, and the reaction was quenched with 50 mL of water. The mixture was extracted three times with 50 mL of ethyl acetate each time. The organic phase was concentrated to dryness. The residue was adsorbed onto silica gel and purified by column chromatography (eluent: petroleum ether/ethyl acetate (V/V)=50:1) to obtain 2-bromo-6-fluoro-4-isobutylbenzonitrile (1.43 g, yield: 62%).

LC-MS, M/Z (ESI): 256.0 [M+H]⁺

### Step 3: Synthesis of 2-fluoro-6-formyl-4-isobutylbenzonitrile

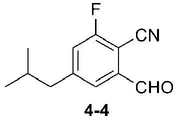

Under a nitrogen atmosphere at -40°C, isopropylmagnesium chloride (1.3 M, 5.2 mL) was added to a solution of 2-bromo-6-fluoro-4-isobutylbenzonitrile (1.43 g, 5.61 mmol) in anhydrous tetrahydrofuran (30 mL). After the mixture reacted at -40°C for 3 hours, DMF (1 mL) was added to the reaction system. Then, the reaction solution was warmed to room temperature and reacted for 1 hour. The reaction was quenched with 50 mL of ice water. The mixture was extracted three times with 50 mL of ethyl acetate each time. The organic phase was washed five times with 50 mL of water each time and concentrated to dryness. The residue was adsorbed onto silica gel and purified by column chromatography (eluent: petroleum ether/ethyl acetate (V/V)=10:1) to obtain 2-fluoro-6-formyl-4-isobutylbenzonitrile (700 mg, yield: 61%).

LC-MS, M/Z (ESI): 206.1 [M+H]⁺

### Step 4: Synthesis of 2-(difluoromethyl)-6-fluoro-4-isobutylbenzonitrile

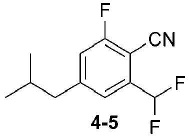

Under a nitrogen atmosphere at 0°C, diethylaminosulfur trifluoride (1.05 g, 6.54 mmol) was added to a solution of 2-fluoro-6-formyl-4-isobutylbenzonitrile (670 mg, 3.26 mmol) in anhydrous dichloromethane (20 mL). Then, the mixture reacted at room temperature for 3 hours. The reaction was quenched with 50 mL of ice water. The mixture was extracted three times with 50 mL of dichloromethane each time. The organic phase was concentrated to dryness. The residue was adsorbed onto silica gel and purified by column chromatography (eluent: petroleum ether/ethyl acetate (V/V)=20:1) to obtain 2-(difluoromethyl)-6-fluoro-4-isobutylbenzonitrile (520 mg, yield: 70%).

LC-MS, M/Z (ESI): 228.1 [M+H]⁺

### Step 5: Synthesis of (S)-2-(difluoromethyl)-4-isobutyl-6-(3-methyl-4-(pyridazin-3-ylmethyl)piperazin-1-yl)benzonitrile

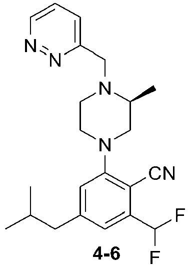

Under a nitrogen atmosphere, anhydrous DMF (5 mL) and N,N-diisopropylethylamine (0.87 mL, 5 mmol) were added to a mixture of (S)-3-((2-methylpiperazin-1-yl)methyl)pyridazine hydrochloride solid (240 mg, 1.0 mmol) and 2-(difluoromethyl)-6-fluoro-4-isobutylbenzonitrile (227 mg, 1.0 mmol). Then, the mixture reacted at 80°C for 16 hours. After the reaction solution was cooled to room temperature, the reaction was quenched with 50 mL of water. The mixture was extracted three times with 50 mL of ethyl acetate each time. The organic phase was washed five times with 50 mL of water each time and concentrated to dryness. The residue was adsorbed onto silica gel and purified by column chromatography (eluent: petroleum ether/ethyl acetate (V/V)=1:1) to obtain (S)-2-(difluoromethyl)-4-isobutyl-6-(3-methyl-4-(pyridazin-3-ylmethyl)piperazin-1-yl)benzonitrile (70 mg, yield: 17.5%).

LC-MS, M/Z (ESI): 400.2 [M+H]⁺

### Step 6: Synthesis of (S)-3-((4-(3-(difluoromethyl)-5-isobutyl-2-(2H-tetrazol-5-yl)phenyl)-2-methylpiperazin-1-yl)methyl)pyridazine

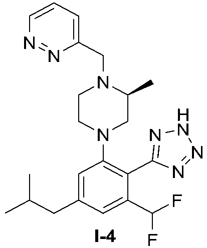

(S)-2-(difluoromethyl)-4-isobutyl-6-(3-methyl-4-(pyridazin-3-ylmethyl)piperazin-1-yl)benzonitrile (70 mg, 0.175 mmol), sodium azide (91.2 mg, 1.4 mmol), tributyltin chloride (0.379 mL, 1.4 mmol), and anhydrous toluene (1.5 mL) were added to a high-temperature and high-pressure resistant autoclave reactor. The reaction solution was bubbled with nitrogen for 1 minute using a nitrogen balloon. Then, the autoclave reactor was sealed, and the mixture reacted at 140°C for 12 hours. After the reaction system was cooled to room temperature, 4 ml of 10% aqueous sodium hydroxide solution and 4 ml of water were added to the reaction solution. The mixture was extracted twice with 30 ml of dichloromethane each time. The pH of the aqueous phase was adjusted to approximately 6 with concentrated hydrochloric acid, and then the aqueous phase was extracted twice with 30 mL of dichloromethane each time. The combined organic phase was dried over anhydrous sodium sulfate and concentrated to dryness. The residue was adsorbed onto silica gel and purified by column chromatography (eluent: ethyl acetate) to obtain (S)-3-((4-(3-(difluoromethyl)-5-isobutyl-2-(2H-tetrazol-5-yl)phenyl)-2-methylpiperazin-1-yl)methyl)pyridazine (30 mg, yield: 39%).

LC-MS, M/Z (ESI): 443.2 [M+H]⁺

¹H NMR (400 MHz, DMSO-d6) δ 9.12-9.06 (m, 1 H), 7.67-7.60 (m, 2 H), 7.23 (s, 1 H), 7.20 (s, 1 H), 6.88-6.55 (m, 1 H), 4.05 (d, 1 H), 3.60 (d, 1 H), 2.75-2.63 (m, 3 H), 2.56 (d, 2 H), 2.50 (m, 2H), 2.39-2.29 (m, 1 H), 2.14-2.03 (m, 1 H),1.94-1.83 (m, 1 H), 0.88 (d, 6 H), 0.85 (d, 3 H).

### Example 2: Preparation of Target Compound I-5

### (S)-3-((4-(3-(difluoromethoxy)-5-isobutyl-2-(2H-tetrazol-5-yl)phenyl)-2-methylpiperazin-1-yl)methyl)pyridazine

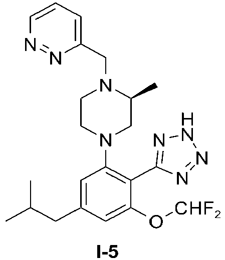

The synthetic scheme of Target Compound **I-5** is as follows:

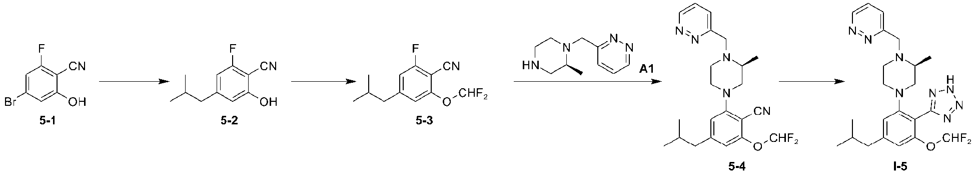

### Step 1: Synthesis of 2-fluoro-6-hydroxy-4-isobutylbenzonitrile

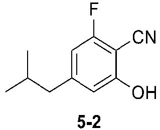

[1,1'-bis(diphenylphosphino)ferrocene]palladium(II) dichloride (1.68 g, 2.3 mmol) and potassium carbonate (9.6 g, 69.3 mmol) were added to a solution of 4-bromo-2-fluoro-6-hydroxybenzonitrile (5 g, 23.1 mmol) and isobutylboronic acid (5.88 g, 57.7 mmol) in dioxane (50 mL). The reaction system was purged with argon three times, and then the mixture reacted at 110°C for 18 hours under an argon atmosphere. The reaction solution was poured into water (200 mL), and the mixture was extracted three times with 100 mL of ethyl acetate each time. The organic phase was washed twice with 100 mL of saturated brine each time, dried over anhydrous sodium sulfate, filtered, and concentrated to obtain a crude product. The crude product was purified by column chromatography (petroleum ether/ethyl acetate (V/V)=1/0 to 0/1) to obtain the product 2-fluoro-6-hydroxy-4-isobutylbenzonitrile (3.2 g, yield 71.5%).

LC-MS, M/Z (ESI): 194.3 [M+H]⁺

### Step 2: Synthesis of 2-(difluoromethoxy)-6-fluoro-4-isobutylbenzonitrile

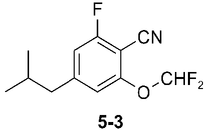

At -78°C, a potassium hydroxide solution (9.3 g, 166 mmol, dissolved in 32 mL water) was added to a solution of 2-fluoro-6-hydroxy-4-isobutylbenzonitrile (3.2 g, 16.6 mmol) in acetonitrile (32 mL), and then diethyl bromodifluoromethylphosphonate (8.9 g, 33.2 mmol) was slowly dropwise added thereto. The mixture was slowly warmed to room temperature and stirred for 18 hours at room temperature. The reaction solution was poured into water (100 mL), and the mixture was extracted three times with 50 mL of ethyl acetate each time. The organic phase was washed twice with 20 mL of saturated brine each time, dried over anhydrous sodium sulfate, filtered, and concentrated to obtain a crude product. The crude product was purified by column chromatography (petroleum ether/ethyl acetate (V/V)=1/0 to 0/1) to obtain the product 2-(difluoromethoxy)-6-fluoro-4-isobutylbenzonitrile (1.6 g, yield 40%).

### Step 3: Synthesis of (S)-2-(difluoromethoxy)-4-isobutyl-6-(3-methyl-4-(pyridazin-3-methyl)piperazin-1-yl)benzonitrile

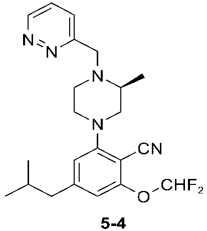

(S)-3-((2-methylpiperazin-1-yl)methyl)pyridazine (1.9 g, 9.9 mmol) and N,N-diisopropylethylamine (2.6 g, 19.8 mmol) were added to a solution of 2-(difluoromethoxy)-6-fluoro-4-isobutylbenzonitrile (1.6 g, 6.6 mmol) in N,N-dimethylformamide (16 mL), and the mixture was stirred at 80°C for 18 hours. The reaction solution was poured into water (100 mL) and extracted three times with 50 mL of ethyl acetate each time. The organic phase was washed twice with 50 mL of saturated brine each time, dried over anhydrous sodium sulfate, filtered, and concentrated to obtain a crude product. The crude product was purified by column chromatography (petroleum ether/ethyl acetate (V/V)=1/0 to 0/1) to obtain the product (S)-2-(difluoromethoxy)-4-isobutyl-6-(3-methyl-4-(pyridazin-3-methyl)piperazin-1-yl)benzonitrile (900 mg, yield 33%).

LC-MS, M/Z (ESI): 416.3 [M+H]⁺

### Step 4: Synthesis of (S)-3-((4-(3-(difluoromethoxy)-5-isobutyl-2-(2H-tetrazol-5-yl)phenyl)-2-methylpiperazin-1-yl)methyl)pyridazine

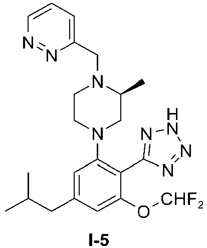

(S)-2-(difluoromethoxy)-4-isobutyl-6-(3-methyl-4-(pyridazin-3-methyl)piperazin-1-yl)benzonitrile (300 mg, 0.7 mmol), sodium azide (364 mg, 5.6 mmol), and tributyltin chloride (1.8 g, 5.6 mmol) were dissolved in toluene (3 mL). The solution was poured into a stainless steel autoclave liner, sealed, and reacted at 140°C for 6 hours. The reaction solution was slowly poured into water (50 mL), and the mixture was extracted three times with 20 mL of ethyl acetate each time. The organic phase was washed twice with 10 mL of saturated brine each time, dried over anhydrous sodium sulfate, filtered, and concentrated to obtain a crude product. The crude product was purified by reverse-phase preparative chromatography (column: Phenomenex Synergi C18, 100×25 mm×4 µm; solvent: A=water+0.1 vol% formic acid (99%), B=acetonitrile; gradient: 5% to 95%, 7 minutes) to obtain the product (S)-3-((4-(3-(difluoromethoxy)-5-isobutyl-2-(2H-tetrazol-5-yl)phenyl)-2-methylpiperazin-1-yl)methyl)pyridazine (42 mg, purity 96.86%, yield 12.7%).

¹H NMR (400 MHz, DMSO-d6) δ 9.10 (dd, 1H), 7.68-7.60 (m, 2H), 7.11 (t, 1H), 6.90 (s, 1H), 6.85 (s, 1H), 4.04 (d, 1H), 3.61 (d, 1H), 2.81-2.65 (m, 3H), 2.55-2.50 (m, 2H), 2.47 (s, 2H), 2.38-2.30 (m, 1H), 2.13-2.05 (m, 1H), 1.93-1.83 (m, 1H), 0.87 (dd, 9H).

LC-MS, M/Z (ESI): 459.3 [M+H]⁺

### Example 3: Preparation of Target Compound I-6

### (S)-3-((4-(5-isobutyl-2-(2H-tetrazol-5-yl)-3-(trifluoromethoxy)phenyl)-2-methylpiperazin-1-yl)methyl)pyridazine

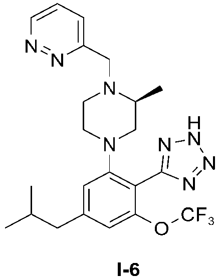

The synthetic scheme of Target Compound **I-6** is as follows:

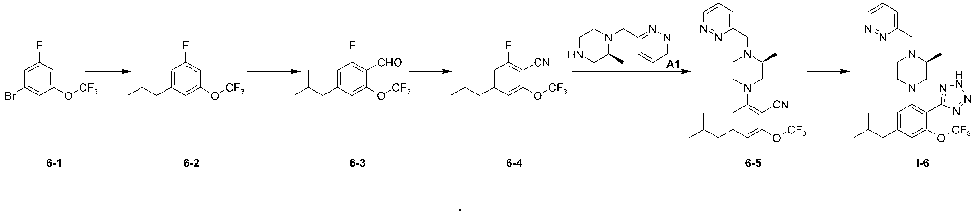

### Step 1: Synthesis of 1-fluoro-3-isobutyl-5-(trifluoromethoxy)benzene

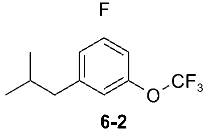

[1,1'-bis(diphenylphosphino)ferrocene]palladium(II) dichloride (585 mg, 0.8 mmol) and potassium carbonate (3.2 g, 23.1 mmol) were added to a solution of 1-bromo-3-fluoro-5-(trifluoromethoxy)benzene (2 g, 7.7 mmol) and isobutylboronic acid (1.57 g, 15.4 mmol) in dioxane (20 mL). Then, the reaction system was purged with nitrogen three times, and the mixture was stirred at 110°C for 18 hours. The reaction solution was poured into water (100 mL), and the mixture was extracted three times with 30 mL of ethyl acetate each time. The organic phase was washed twice with 20 mL of saturated brine each time, dried over anhydrous sodium sulfate, filtered, and concentrated to obtain a crude product. The crude product was purified by column chromatography (petroleum ether/ethyl acetate (V/V)=1/0 to 1/1) to obtain the product 1-fluoro-3-isobutyl-5-(trifluoromethoxy)benzene (1 g, yield 54.8%).

### Step 2: Synthesis of 2-fluoro-4-isobutyl-6-(trifluoromethoxy)benzaldehyde

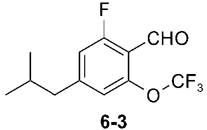

1-fluoro-3-isobutyl-5-(trifluoromethoxy)benzene (1 g, 4.2 mmol) was dissolved in tetrahydrofuran (10 mL), and the reaction system was purged with nitrogen three times. Then, lithium diisopropylamide (5 mL, 5 mmol, 1M in tetrahydrofuran) was slowly added dropwise to the reaction solution at -78°C, and the mixture was stirred for 0.5 hours. Then, N,N-dimethylformamide (3 mL) was slowly added dropwise at the same temperature, and the mixture was stirred for 2 hours. The reaction solution was slowly added dropwise to saturated ammonium chloride solution (50 mL), and the mixture was extracted three times with 20 mL of ethyl acetate each time. The organic phase was washed twice with 20 mL of saturated brine each time, dried over anhydrous sodium sulfate, filtered, and concentrated to obtain a crude product. The crude product was purified by column chromatography (petroleum ether/ethyl acetate (V/V)=1/0 to 1/1) to obtain the product 2-fluoro-4-isobutyl-6-(trifluoromethoxy)benzaldehyde (0.7 g, yield 62.6%).

### Step 3: Synthesis of 2-fluoro-4-isobutyl-6-(trifluoromethoxy)benzonitrile

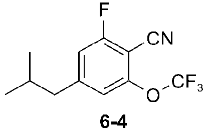

Iodine (3.3 g, 13 mmol) was added to a solution of 2-fluoro-4-isobutyl-6-(trifluoromethoxy)benzaldehyde (700 mg, 2.6 mmol) in tetrahydrofuran (7 mL), and ammonia solution (7 mL) was slowly added dropwise thereto at 0°C. Then, the mixture was stirred at 25°C for 18 hours. The reaction solution was slowly added dropwise to water (50 mL), and the mixture was extracted three times with 20 mL of ethyl acetate each time. The organic phase was washed twice with 20 mL of saturated sodium sulfite solution each time and twice with 20 mL of saturated brine each time, dried over anhydrous sodium sulfate, filtered, and concentrated to obtain a crude product. The crude product was purified by column chromatography (petroleum ether/ethyl acetate (V/V)=1/0 to 1/1) to obtain the product 2-fluoro-4-isobutyl-6-(trifluoromethoxy)benzonitrile (0.6 g, yield 86.7%).

### Step 4: Synthesis of (S)-4-isobutyl-2-(3-methyl-4-(pyridazin-3-ylmethyl)piperazin-1-yl)-6-(trifluoromethoxy)benzonitrile

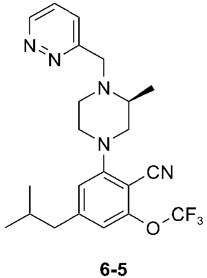

(S)-3-((2-methylpiperazin-1-yl)methyl)pyridazine (231 mg, 1.2 mmol) and N,N-diisopropylethylamine (310 mg, 2.4 mmol) were added to a solution of 2-fluoro-4-isobutyl-6-(trifluoromethoxy)benzonitrile (200 mg, 0.8 mmol) in N,N-dimethylformamide (2 mL), and the mixture was stirred at 80°C for 18 hours. The reaction solution was slowly added dropwise to water (50 mL), and the mixture was extracted three times with 20 mL of ethyl acetate each time. The organic phase was washed five times with 20 mL of water each time and twice with 20 mL of saturated brine each time, dried over anhydrous sodium sulfate, filtered, and concentrated to obtain a crude product. The crude product was purified by column chromatography (petroleum ether/ethyl acetate (V/V)=1/0 to 0/1) to obtain the product 2-fluoro-4-isobutyl-6-(trifluoromethoxy)benzonitrile (150 mg, yield 45.2%).

LC-MS, M/Z (ESI): 434.3 [M+H]⁺

### Step 5: Synthesis of: (S)-3-((4-(5-isobutyl-2-(2H-tetrazol-5-yl)-3-(trifluoromethoxy)phenyl)-2-methylpiperazin-1-yl)methyl)pyridazine

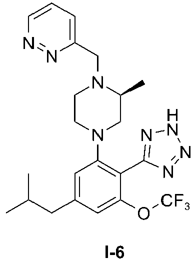

2-fluoro-4-isobutyl-6-(trifluoromethoxy)benzonitrile (150 mg, 0.3 mmol), sodium azide (156 mg, 2.4 mmol), and tributyltin chloride (781 mg, 2.4 mmol) were dissolved in toluene (1.5 mL). The solution was poured into a stainless steel autoclave liner, sealed, and reacted at 140°C for 12 hours. The reaction solution was slowly poured into water (50 mL), and the mixture was extracted three times with 20 mL of ethyl acetate each time. The organic phase was washed twice with 10 mL of saturated brine each time, dried over anhydrous sodium sulfate, filtered, and concentrated to obtain a crude product. The crude product was purified by reverse-phase preparative chromatography (column: Phenomenex Synergi C18, 100×25 mm×4 µm; solvent: A=water+0.1 vol% formic acid (99%), B=acetonitrile; gradient: 5% to 95%, 7 minutes) to obtain the product (S)-3-((4-(5-isobutyl-2-(2H-tetrazol-5-yl)-3-(trifluoromethoxy)phenyl)-2-methylpiperazin-1-yl)methyl)pyridazine (61 mg, purity 99.47%, yield 37%).

¹H NMR (400 MHz, CDCl₃) δ 9.01 (d, 1H), 7.72-7.65 (m, 1H), 7.49 (dd, 1H), 6.90 (s, 1H), 6.83 (s, 1H), 4.20 (d, 1H), 3.68 (d, 1H), 2.85-2.75 (m, 3H), 2.61-2.49 (m, 5H), 2.36 (dd, 1H), 1.88 (dt, 1H), 0.99 (d, 3H), 0.93 (d, 6H).

LC-MS, M/Z (ESI): 477.3 [M+H]⁺

### Example 4: Preparation of Target Compound I-7

### (2S)-4-(3-(difluoromethyl)-2-(3H-1,2,3,4-tetrazol-5-yl)-5-isobutylphenyl)-2-methyl-1-((5-methyl-1,3,4-thiadiazol-2-yl)methyl)piperazine

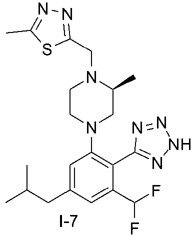

The synthetic scheme of Target Compound **I-7** is as follows:

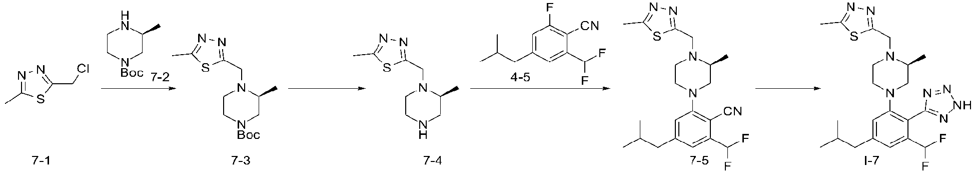

### Step 1: Synthesis of: tert-butyl (3S)-3-methyl-4-((5-methyl-1,3,4-thiadiazol-2-yl)methyl)piperazine-1-carboxylate

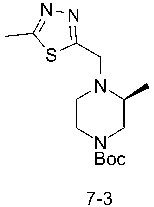

Under a nitrogen atmosphere, anhydrous DMF (15 mL) was added to a mixture of 2-(chloromethyl)-5-methyl-1,3,4-thiadiazole (440 mg, 3 mmol), (S)-4-N-tert-butoxycarbonyl-2-methylpiperazine (600 mg, 3 mmol), and cesium carbonate (2.44 g, 25 mmol). Then, the mixture reacted at 50°C for 16 hours. After the reaction solution was cooled to room temperature, the reaction was quenched with water. The mixture was extracted three times with 30 mL of ethyl acetate each time and washed five times with 40 mL of water each time. The organic phase was collected and concentrated to dryness. The residue was adsorbed onto silica gel and purified by column chromatography (eluent: petroleum ether/ethyl acetate=1/2) to obtain tert-butyl (3S)-3-methyl-4-((5-methyl-1,3,4-thiadiazol-2-yl)methyl)piperazine-1-carboxylate (500 mg, yield: 53.2%).

LC-MS, M/Z (ESI): 313.4 [M+H]⁺

### Step 2: Synthesis of (2S)-2-methyl-5-((2-methylpiperazin-1-yl)methyl)-1,3,4-thiadiazole

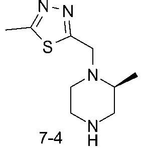

Under a nitrogen atmosphere, 4 M hydrochloric acid in 1,4-dioxane (5 mL) was added to a solution of tert-butyl (3S)-3-methyl-4-((5-methyl-1,3,4-thiadiazol-2-yl)methyl)piperazine-1-carboxylate (312 mg, 1.0 mmol) in dichloromethane (5 mL). The mixture reacted at room temperature for 2 hours. The reaction solvent was concentrated under reduced pressure to obtain (2S)-2-methyl-5-((2-methylpiperazin-1-yl)methyl)-1,3,4-thiadiazole (hydrochloride, 176 mg), which was directly used in the next reaction.

LC-MS, M/Z (ESI): 213.3 [M+H]⁺

### Step 3: Synthesis of: (3S)-2-(difluoromethyl)-4-isobutyl-6-(3-methyl-4-((5-methyl-1,3,4-thiadiazol-2-yl)methyl)piperazin-1-yl)benzonitrile

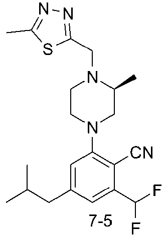

Under a nitrogen atmosphere, anhydrous DMF (4 mL) and N,N-diisopropylethylamine (0.73 mL, 3.36 mmol) were added to a mixture of (2S)-2-methyl-5-((2-methylpiperazin-1-yl)methyl)-1,3,4-thiadiazole (hydrochloride, 176 mg, 0.84 mmol) and 2-(difluoromethyl)-6-fluoro-4-isobutylbenzonitrile (190 mg, 0.84 mmol). Then, the mixture reacted at 80°C for 16 hours. After the reaction solution was cooled to room temperature, the reaction was quenched with water. The mixture was extracted three times with 30 mL of ethyl acetate each time and washed five times with 40 mL of water each time. The organic phase was collected and concentrated to dryness. The residue was adsorbed onto silica gel and purified by column chromatography (eluent: petroleum ether/ethyl acetate=1/1) to obtain (3S)-2-(difluoromethyl)-4-isobutyl-6-(3-methyl-4-((5-methyl-1,3,4-thiadiazol-2-yl)methyl)piperazin-1-yl)benzonitrile (150 mg, yield: 42%).

LC-MS, M/Z (ESI): 420.5 [M+H]⁺

### Step 4: Synthesis of (2S)-4-(3-(difluoromethyl)-2-(3H-1,2,3,4-tetrazol-5-yl)-5-isobutylphenyl)-2-methyl-1-((5-methyl-1,3,4-thiadiazol-2-yl)methyl)piperazine

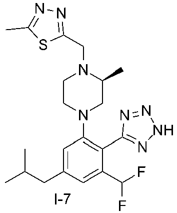

(S)-2-(difluoromethyl)-4-isobutyl-6-(3-methyl-4-((5-methyl-1,3,4-thiadiazol-2-yl)methyl)piperazin-1-yl)benzonitrile (150 mg, 0.36 mmol), sodium azide (187 mg, 2.88 mmol), tributyltin chloride (0.77 mL, 2.88 mmol), and anhydrous toluene (3 mL) were added to a high-temperature and high-pressure resistant metallic autoclave reactor. The reaction solution was bubbled with nitrogen for 1 minute using a nitrogen balloon. Then, the autoclave reactor was sealed, and the mixture reacted at 140°C for 12 hours. After the reaction system was cooled to room temperature, 4 mL of 10% aqueous sodium hydroxide solution and 4 mL of water were added to the reaction solution. The mixture was extracted twice with 30 mL of dichloromethane each time. The pH of the aqueous phase was adjusted to approximately 6 with concentrated hydrochloric acid, and the aqueous phase was extracted twice with 30 mL of dichloromethane each time. The combined organic phase was dried over anhydrous sodium sulfate and concentrated to dryness. The residue was adsorbed onto silica gel and purified by column chromatography (eluent: ethyl acetate) to obtain (2S)-4-(3-(difluoromethyl)- 2-(3H-1,2,3,4-tetrazol-5-yl)-5-isobutylphenyl)-2-methyl-1-((5-methyl-1,3,4-thiadiazol-2-yl)methyl)piperazine (50 mg, yield: 30%).

LC-MS, M/Z (ESI): 463.6 [M+H]⁺

¹H NMR (400 MHz, DMSO*_{d6}*) δ 7.25 (s, 1 H), 7.23 (s, 1 H), 6.88-6.55 (m, 1 H), 4.08 (d, 1 H), 3.77 (d, 1 H), 2.69 (s, 2 H), 2.66 (s, 3 H), 2.57 (d, 3 H), 2.5-2.4 (m, 2 H), 2.38-2.29 (m, 1H), 2.22-2.12 (m, 1 H), 1.95-1.83 (m, 1 H), 0.89 (s, 3 H), 0.87 (s, 3 H), 0.82 (d, 3 H).

### Example 5: Preparation of Target Compound I-8

### (2S)-4-(3-((difluoromethyl)oxy)-2-(3H-1,2,3,4-tetrazol-5-yl)-5-isobutylphenyl)-2-methyl-1-((5-methyl-1,3,4-thiadiazol-2-yl) methyl) piperazine

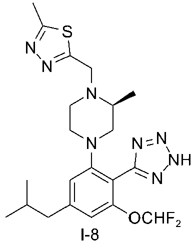

The synthetic scheme of Target Compound **I-8** is as follows:

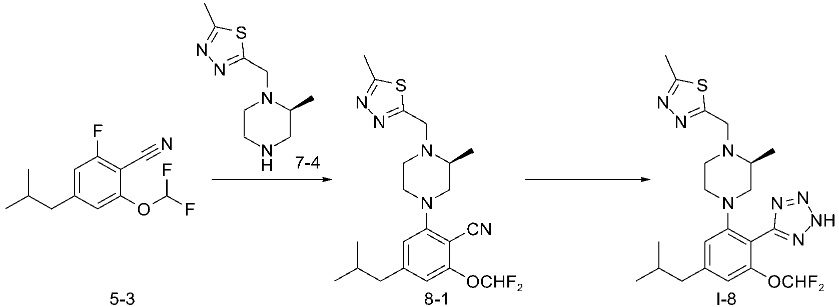

### Step 1: Synthesis of (3S)-2-(difluoromethoxy)-4-isobutyl-6-(3-methyl-4-((5-methyl-1,3,4-thiadiazol-2-yl)methyl)piperazin-1-yl)benzonitrile

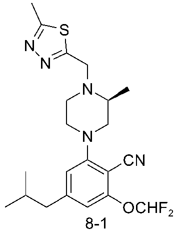

Potassium carbonate (438.74 mg, 3.18 mmol) and 2-(difluoromethoxy)-6-fluoro-4-isobutylbenzonitrile (121 mg, 423.9 µmol) were added to a solution of (2S)-2-methyl-5-((2-methylpiperazin-1-yl)methyl)-1,3,4-thiadiazole (135 mg, 635.85 µmol) in DMF (5 mL). The reaction solution was stirred at 85°C for 4 hours. LCMS indicated complete consumption of the starting materials. Water (30 mL) was added to the reaction solution, and the mixture was extracted three times with 30 mL of ethyl acetate each time and washed five times with 30 mL of water each time. The organic layer was collected, dried over anhydrous sodium sulfate, and concentrated. The crude product was purified by silica gel chromatography column and eluted with ethyl acetate (0% to 50%)/petroleum ether to obtain (3S)-2-(difluoromethoxy)-4-isobutyl-6-(3-methyl-4-((5-methyl-1,3,4-thiadiazol-2-yl)methyl)piperazin-1-yl)benzonitrile (200.00 mg, 459.21 µmol, yield: 72.22%).

LC-MS (ESI): 436.2 [M+H]⁺

### Step 2: Synthesis of: (2S)-4-(3-((difluoromethyl)oxy)-2-(3H-1,2,3,4-tetrazol-5-yl)-5-isobutylphenyl)-2-methyl-1-((5-methyl-1,3,4-thiadiazol-2-yl)methyl)piperazine

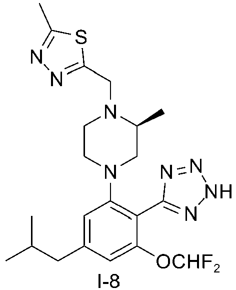

Under a nitrogen atmosphere, a solution of (3S)-2-(difluoromethoxy)-4-isobutyl-6-(3-methyl-4-((5-methyl-1,3,4-thiadiazol-2-yl)methyl)piperazin-1-yl)benzonitrile (180 mg, 413.29 µmol) and tributyltin chloride (1.08 g, 3.31 mmol) in toluene (5 mL) was microwave-heated at 150°C and reacted for 7 hours. LCMS indicated almost complete consumption of the starting materials. The reaction solution was extracted three times with 30 mL of ethyl acetate each time and washed five times with 30 mL of water each time. The organic layer was collected and concentrated. The residue was purified by high-performance liquid chromatography column (acetonitrile/water, 0.1% formic acid) and dried to obtain (2S)-4-(3-((difluoromethyl)oxy)-2-(3H-1,2,3,4-tetrazol-5-yl)-5-isobutylphenyl)-2-methyl-1-((5-methyl-1,3,4-thiadiazol-2-yl)methyl)piperazine (10.10 mg, 21.10 µmol, yield: 5.11%).

LC-MS (ESI): 479.2 [M+H]⁺

¹H NMR (400 MHz, Chloroform-d) δ 6.85 (dd, J=9.8, 1.4 Hz, 2H), 6.60 (t, J=73.9 Hz, 1H), 4.21 (d, J=15.1 Hz, 1H), 3.90 (d, J=15.1 Hz, 1H), 2.84 (dd, J=28.6, 12.4 Hz, 4H), 2.73 (s, 3H), 2.65-2.44 (m, 5H), 1.90 (dt, J=13.5, 6.8 Hz, 1H), 1.09-0.99 (m, 3H), 0.94 (d, J=6.6 Hz, 6H).

**Example 6:** The following compounds can be prepared by referring to the preparation methods of the compounds described above.

| **Structural Formula** | **Name** | **LC-MS, M/Z (ESI)** |
|---|---|---|
| 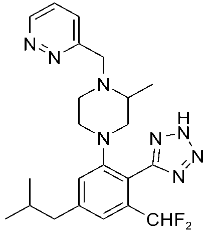 | 3-((4-(5-isobutyl-2-(2H-tetrazol-5-yl)-3-(difluoromethyl)phenyl)-2-methylpiperazin-1-yl)methyl)pyridazine | 443.2 |
| 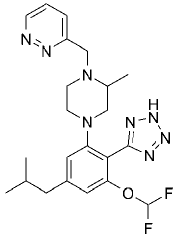 | 3-((4-(3-(difluoromethoxy)-5-isobutyl-2-(2H-tetrazol-5-yl)phenyl)-2-methylpiperazin-1-yl)methyl)pyridazine | 459.2 |
| 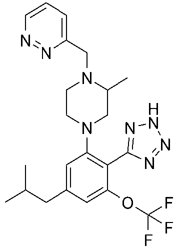 | 3-((4-(5-isobutyl-2-(2H-tetrazol-5-yl)-3-(trifluoromethoxy)phenyl)-2-methylpiperazin-1-yl)methyl)pyridazine | 477.2 |
| 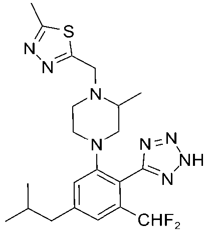 | 4-(3-(difluoromethyl)-2-(3H-1,2,3,4-tetrazol-5-yl)-5-isobutylphenyl)-2-methyl-1-((5-methyl-1,3,4-thiadiazol-2-yl)methyl)piperazine | 463.2 |
| 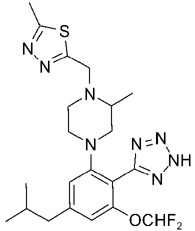 | 4-(3-((difluoromethyl)oxy)-2-(3H-1,2,3,4-tetrazol-5-yl)-5-isobutylphenyl)-2-methyl-1-((5-methyl-1,3,4-thiadiazol-2-yl)methyl)piperazine | 479.2 |
| 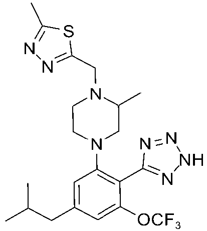 | 4-(2-(3H-1,2,3,4-tetrazol-5-yl)-5-isobutyl-3-((trifluoromethyl)oxy)phenyl)-2-methyl-1-((5-methyl-1,3,4-thiadiazol-2-yl)methyl)piperazine | 497.2 |
| 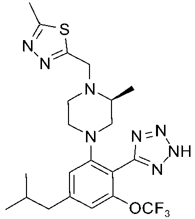 | (2S)-4-(2-(3H-1,2,3,4-tetrazol-5-yl)-5-isobutyl-3-((trifluoromethyl)oxy)phenyl)-2-methyl-1-((5-methyl-1,3,4-thiadiazol-2-yl)methyl)piperazine | 497.2 |

In the test examples of the present disclosure, Control Compound I was prepared by the method referring to Patent WO2023006893A1. The Control Compound I has a structure represented by:

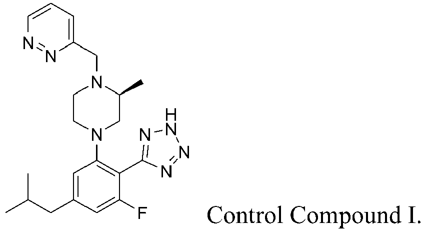

### Bioassays

The following test methods can be adopted.

### Test Example 1: Binding Assay of Compounds to AT2R

The assay was performed in accordance with the manufacturer instructions of Angiotensin AT2 Receptor Ligand Binding Assay Kit (#C1TT1AT2, Cisbio). First, a 10 mM compound stock solution was diluted in a gradient at a 5× dilution ratio (including 10 concentrations, each with two replicates). 160 nL of compounds at different concentrations was added to a 384-well plate. 40 µL of 1× TLB was added to each well, and the mixture was shaken at room temperature for 15 minutes. A 15 mL centrifuge tube added with 5 mL of 1×TLB was prepared in advance. The frozen labeled cells were thawed in a water bath at 37°C (for 1 to 2 minutes), and the thawed cells were quickly transferred to the above 15 mL centrifuge tube. The mixture was mixed well and centrifuged at 1,000 g at room temperature for 5 minutes. The supernatant was removed, and the cells were resuspended by adding 2.7 mL 1× TLB. A new 384-well plate was taken, and 10 µL of mixed cells were added into the corresponding wells according to the assay design. 5 µL of 4× compound solution and 5 µL of 4× Tag-lite red fluorescent labeled ligand were added to each well. After incubation at room temperature for 1 hour, data was read using EnVision with an HTRF mode. The excitation light intensities at 665nM and 615nM in each well were read respectively, and the ratio was calculated (Ratio=A665nM/B615nM). The IC₅₀ value was calculated by means of GraphPad Prism8 software, where X represents logarithmic value of compound concentration; and Y represents ratio of A665nM/B615nM.

**[Table 1] Binding activity of compounds to AT2R**

| Compound | AT2R IC₅₀ (nM) |
|---|---|
| Control Compound I | 12 |
| I-4 | 4.1 |
| I-5 | 2.4 |
| I-6 | 3.6 |
| I-7 | 8.0 |
| I-8 | 2.5 |

The experimental results showed that in the binding activity assay of the test compounds to AT2R, the compounds of the present disclosure exhibited a stronger binding affinity to AT2R, which were superior to Control Compound I.

### Test Example 2: Pharmacokinetic Test in Rats

For the pharmacokinetic test of rats, 3 male SD rats (180 g to 240 g) were employed and fasted overnight. The rats were orally administered a dose of 10 mg/kg by gavage. Blood was collected before the administration, and at 15 minutes, 30 minutes, 1 hour, 2 hours, 4 hours, 6 hours, 8 hours, and 24 hours after the administration. The blood samples were centrifuged at 8,000 rpm and at 4°C for 6 minutes, and plasma was collected and stored at -20°C. The plasma at each time point was taken and mixed with 3 to 5 volumes of an acetonitrile solution containing an internal standard. The mixture was vortexed for 1 minute, and centrifuged at 13,000 rpm and at 4°C for 10 minutes. The supernatant was collected and mixed with 3 volumes of water. An appropriate amount of the mixture was taken for LC-MS/MS analysis. The main pharmacokinetic parameters were analyzed by a non-compartmental model using WinNonlin 7.0 software.

**[Table 2] Results of Pharmacokinetic Test in Rats**

| Compound | Pharmacokinetic Parameters in Rats | |
|---|---|---|
| | Oral Administration by Gavage | |
| | Cmax (ng/mL) | AUC₀₋ₜ (h·ng/mL) |
| Control Compound I | 3,240 | 10,028 |
| I-4 | 4,377 | 14,072 |

The experimental results showed that the compounds of the present disclosure exhibited excellent pharmacokinetic properties in rats, which were superior to Control Compound I.

### Test Example 3: Pharmacokinetic Test in Mice

For the pharmacokinetic test of mice, 3 male ICR mice (20 g to 30 g) were employed and fasted overnight. The mice were orally administered a dose of 10 mg/kg by gavage. Blood was collected before the administration, and at 5 minutes, 15 minutes, 30 minutes, 1 hour, 2 hours, 4 hours, 6 hours, 8 hours, and 24 hours after the administration. The blood samples were centrifuged at 6,000 g and at 2°C to 8°C for 3 minutes, and plasma was collected and stored at -20°C. The plasma at each time point was taken and mixed with 10 volumes of a 50% methanol in acetonitrile solution containing an internal standard. The mixture was vortexed for 5 minutes, and centrifuged at 4,000 rpm and at 4°C for 10 minutes. The supernatant was collected and mixed with 1 volume of water. An appropriate amount of the mixture was taken for LC-MS/MS analysis. The main pharmacokinetic parameters were analyzed by a non-compartmental model using WinNonlin 7.0 software.

The experimental results showed that the compounds of the present disclosure exhibited excellent pharmacokinetic properties in mice.

### Test Example 4: Pharmacokinetic Test in Canines

For the pharmacokinetic test of canines, 3 male Beagle canines (10 kg to 12 kg) were employed and fasted overnight. The canines were orally administered a dose of 5 mg/kg by gavage. Blood was collected before the administration, and at 5 minutes, 15 minutes, 30 minutes, 1 hour, 2 hours, 4 hours, 6 hours, 8 hours, and 24 hours after the administration. The blood samples were centrifuged at 6,000 g and at 2°C to 8°C for 3 minutes, and plasma was collected and stored at -20°C. The plasma at each time point was taken and mixed with 10 volumes of a 50% methanol in acetonitrile solution containing an internal standard. The mixture was vortexed for 5 minutes, and centrifuged at 4,000 rpm and at 4°C for 5 minutes. The supernatant was collected and mixed with 1 volume of water. An appropriate amount of the mixture was taken for LC-MS/MS analysis. The main pharmacokinetic parameters were analyzed by a non-compartmental model using WinNonlin 7.0 software.

**[Table 3] Results of Pharmacokinetic Test in Canines**

| Compound | Pharmacokinetic Parameters in Canines | |
|---|---|---|
| | Cmax (ng/mL) | AUC₀₋ₜ (h·ng/mL) |
| Control Compound I | 850 | 1,295 |
| I-5 | 1,321 | 2,294 |

The experimental results showed that the compounds of the present disclosure exhibited excellent pharmacokinetic properties in canines, which were superior to Control Compound I.

### Test Example 5: Effects of Compounds on hERG Channel Currents Determined by Manual Patch-Clamp Technique

A manual patch-clamp assay was conducted using an HEK-293 cell line stably expressing an hERG potassium channel. hERG potassium channel cells were purchased from Creacell Co., Ltd. (catalog No.: A-0320). Final concentrations of test compounds were freshly prepared on the day of the assay, and then the test compounds were dissolved in an extracellular solution. The extracellular solution (mM) was: 40 mM NaCl, 3.5 mM KCl, 1 mM MgCl₂·6H₂O, 2 mM CaCl₂·2H₂O, 10 mM D-Glucose, 10 mM HEPES, 1.25 mM NaH₂PO₄·2H₂O; pH 7.4 (titrated with NaOH). An intracellular solution (mM) was: 20 mM KCl, 115 mM K-Aspartic, 1 mM MgCl₂·6H₂O, 5 mM EGTA, 10 mM HEPES, 2 mM Na₂-ATP; pH 7.4 (titrated with KOH).

Electrophysiological Assay: After whole-cell patch-clamp formation, the cell membrane potential was clamped at -80 mV. The holding potential was depolarized to -50 mV from -80 mV and maintained for 0.5 seconds (for leak current detection), then stepped to 30 mV for 2.5 seconds, and then rapidly repolarized back to -50 mV for 4 seconds to elicit a tail current of the hERG channel. Data was collected repeatedly every 10 seconds to observe the effects of the drug on hERG tail current (Peak tail current_{compound}). A stimulation at -50 mV for 0.5 seconds was used for the leak current detection. Drug administration was initiated after the hERG current recorded in the whole-cell configuration stabilized. After each drug concentration was applied for 5 minutes (or until the current stabilized), the next concentration was tested. One or more concentrations were tested for each test compound. A current detected in each cell in a compound-free extracellular solution (Peak tail current_{Control}) served as an internal control for the corresponding cell. For each concentration, independent duplicate measurements were performed using at least two cells. All the electrophysiological assays were conducted at room temperature. The experimental data was collected by an IPA amplifier (Sutter Instrument) and stored in Sutter Patch software.

Data Analysis: The inhibition rate corresponding to each drug concentration was calculated (1-(Peak tail current_{compound}/Peak tail current_{Control})), and the concentration-response curves were fitted using nonlinear regression in GraphPad Prism software. The IC₅₀ value of each compound was calculated using the following equation: Y=1/(1+10^((LogIC₅₀-X)×HillSlope)).

**[Table 4] Results of Inhibitory Effects of Compounds on hERG**

| Compound | IC₅₀ (µM) |
|---|---|
| Control Compound I | 2.8 |
| I-4 | >30 |
| I-5 | 22.8 |

The experimental results showed that the compounds of the present disclosure exhibited a lower risk of hERG inhibition in an *in vitro* system, indicating a more favorable cardiac safety profile compared to Control Compound I.

### Test Example 6: Chronic Constriction Injury (CCI) Model of Sciatic Nerve in Rats

Male SD rats weighing 200 g to 250 g were anesthetized and then fixed in the prone position. The area from the lateral thigh to the femur of the animals was shaved and disinfected, and a 1.5 cm incision was made on the thigh skin. The fascia was dissected, and the biceps femoris muscle was bluntly dissected to expose the sciatic nerve. Approximately 7 mm of the nerve was freed at the main trunk of the sciatic nerve proximal to its trifurcation, and the sciatic nerve was gently dissected. At 2 mm proximal to the trifurcation, the sciatic nerve was ligated with four ligatures of 5-0 suture, each spaced 1 mm apart, resulting in a ligated nerve segment approximately 4 mm to 5 mm in length. The tightness of the ligation was determined by visible slight twitching of the calf muscles or toes when the ligature was tied. The muscle, subcutaneous tissue, and skin were sequentially sutured, and the wound was cleaned and disinfected with iodophor. The skin was sutured and disinfected. On the second day after modeling, the animals were randomly divided into groups with 8 rats per group, and the animals in each group were administered the vehicle or corresponding compound solution via oral gavage. The mechanical pain threshold of each animal was measured using Von-Frey filaments before and after the administration.

Method for Measuring Mechanical Pain Threshold: The test animals were continuously stimulated using Von-Frey filaments on the plantar surface of the hind paw to be tested until the filaments bent, and paw withdrawal response of the animals was observed. The test animals were stimulated with the filaments in the ascending order of gram weight, and each gram weight filament was used to stimulate the animals 5 times consecutively. If fewer than 3 positive responses were observed, the above operation was repeated with the next higher gram weight filament. The filament value was defined as the pain threshold of the animal when 3 or more positive responses were observed for the first time in the test (each animal was tested 3 times and the average was taken). The gram weights of the filaments were 0.6, 1.0, 1.4, 2.0, 4.0, 6.0, 8.0, 10.0, and 15.0, with a cut-off value of 15.0 g. The data of each group was statistically analyzed using GraphPad Prism 8.0, and the statistical method was one-way ANOVA to compare the statistical significance among the groups, where P<0.05 was considered to indicate statistical significance.

The experimental results showed that in the chronic constriction injury model of the sciatic nerve in the rats, the compounds of the present disclosure can significantly ameliorate a reduction in the mechanical pain threshold of the animals induced by the sciatic nerve modeling in the rats, exerting excellent analgesic effects.

Although embodiments of the present disclosure have been shown and described, it would be appreciated that the above embodiments are exemplary and cannot be construed to limiting the present disclosure, and changes, modifications, alternatives, and alterations can be made in the embodiments by those skilled in the art without departing from scope of the present disclosure.

## Claims

1. A compound represented by Formula I, or a tautomer, stereoisomer, or pharmaceutically acceptable salt thereof: 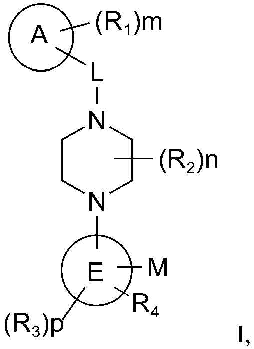 wherein:
ring A is selected from a 5- to 6-membered heterocyclic ring;
R₁ is selected from halogen, hydroxyl, amino, cyano, oxo (=O), C₁ to C₆ alkyl, C₁ to C₆ alkoxy, and C₃ to C₇ cycloalkyl, wherein the C₁ to C₆ alkyl, the C₁ to C₆ alkoxy, and the C₃ to C₇ cycloalkyl are optionally substituted with one to four substituents selected from halogen, hydroxyl, amino, cyano, C₁ to C₆ alkyl, C₁ to C₆ alkoxy, C₃ to C₇ cycloalkyl, and oxo;
m is selected from 0, 1, 2, 3, or 4, and when a plurality of substituents R₁ are present, the plurality of substituents R₁ are the same or different;
L is absent or selected from C₀ to C₆ alkyl-O-Co to C₆ alkyl, C₀ to C₆ alkyl-NH-Co to C₆ alkyl, and C₁ to C₆ alkyl, wherein the C₁ to C₆ alkyl-O-C₁ to C₆ alkyl, the C₀ to C₆ alkyl-NH-Co to C₆ alkyl, and the C₁ to C₆ alkyl are optionally substituted with one to four substituents selected from deuterium, oxo (=O), methyl, ethyl, halogenated methyl, and halogenated ethyl;
R₂ is selected from oxo (=O), C₁ to C₆ alkyl, C₁ to C₆ alkoxy, C₃ to C₇ cycloalkyl, a 5- to 10-membered heterocyclic ring, and a 6- to 10-membered aromatic ring, wherein the C₁ to C₆ alkyl, the C₁ to C₆ alkoxy, and the C₃ to C₇ cycloalkyl are optionally substituted with one, two, three, or four substituents selected from halogen, hydroxyl, cyano, amino, C₁ to C₆ alkyl, C₁ to C₆ alkoxy, and C₃ to C₇ cycloalkyl;
n is selected from 0, 1, 2, 3, 4, 5, 6, 7, or 8, and when a plurality of substituents R₂ are present, the plurality of substituents R₂ are the same or different;
ring E is selected from a benzene ring or a 5- to 6-membered heteroaromatic ring;
R₃ is selected from halogen, hydroxyl, cyano, dimethylamino, C₁ to C₆ alkyl, C₁ to C₆ alkoxy, and C₃ to C₇ cycloalkyl, wherein the C₁ to C₆ alkyl, the C₁ to C₆ alkoxy, and the C₃ to C₇ cycloalkyl are optionally substituted with one to four substituents selected from halogen, hydroxyl, cyano, C₁ to C₆ alkyl, C₁ to C₆ alkoxy, and C₃ to C₇ cycloalkyl;
p is selected from 0, 1, 2, 3, or 4, and when a plurality of substituents R₃ are present, the plurality of substituents R₃ are the same or different;
M is selected from carboxylic group, -CONHSO₂R₅, -SO₂NHCOR₅, tetrazole, or a tetrazole bioisostere, wherein the tetrazole bioisostere comprises phosphoric acid, 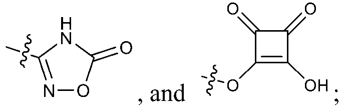
R₄ is selected from -CHF₂, -CH₂F, -(C₀ to C₆ alkyl)-O-(halogenated C₁ to C₆ alkyl), and - (Co to C₆ alkyl)-O-(C₃ to C₄ cycloalkyl), wherein the -(Co to C₆ alkyl)-O-(halogenated C₁ to C₆ alkyl) and the -(Co to C₆ alkyl)-O-(C₃ to C₄ cycloalkyl) are optionally substituted with one to four substituents selected from hydroxyl, amino, C₃ to C₇ heterocycloalkyl, C₁ to C₃ alkyl, C₃ to C₆ cycloalkyl, and C₁ to C₃ alkoxy;
M and R₄ are on the ring E in an ortho relationship; and
R₅ is selected from C₁ to C₈ alkyl, C₃ to C₈ cycloalkyl, C₁ to C₈ alkoxy, and a 5- to 6-membered heterocyclic ring, wherein the C₁ to C₈ alkyl, the C₃ to C₈ cycloalkyl, the C₁ to C₈ alkoxy, and the 5- to 6-membered heterocyclic ring are optionally substituted with one to four substituents selected from halogen, amino, C₃ to C₇ heterocycloalkyl, C₁ to C₃ alkyl, C₃ to C₆ cycloalkyl, and C₁ to C₃ alkoxy.

2. The compound represented by Formula I, or the tautomer, stereoisomer, or pharmaceutically acceptable salt thereof according to claim 1, satisfying one or more of the following conditions a) to d):
a) the ring A is a 5- to 6-membered heteroaromatic ring, 5- to 6-membered heterocycloalkenyl, or 5- to 6-membered heterocycloalkyl, wherein the 5- to 6-membered heteroaromatic ring, the 5- to 6-membered heterocycloalkenyl, or the 5- to 6-membered heterocycloalkyl comprises one, two, or three identical or different heteroatoms, and preferably, the one, two, or three identical or different heteroatoms are selected from N, O, and S;
preferably, the ring A is selected from: furanyl, pyrrolyl, thienyl, oxazolyl, oxadiazolyl, thiazolyl, thiadiazolyl, pyrazolyl, imidazolyl, triazolyl, tetrazolyl, pyridinyl, pyrimidinyl, pyridazinyl, triazinyl, dihydropyrrolyl, dihydropyrazolyl, dihydroimidazolyl, dihydrotriazolyl, dihydrotetrazolyl, dihydrotriazolyl, dihydrotetrazolyl, tetrahydropyridinyl, dihydropyridinyl, tetrahydropyrazinyl, dihydropyrazinyl, tetrahydropyrimidinyl, dihydropyrimidinyl, pyrrolidinyl, pyrazolidinyl, imidazolidinyl, piperidinyl, piperazinyl, hexahydropyridazinyl, and hexahydropyrimidinyl; and
more preferably, the ring A is selected from pyridinyl, pyrimidinyl, pyridazinyl, pyrazinyl, and thiadiazolyl;
b) the ring E is a benzene ring or a 5- to 6-membered heteroaromatic ring, wherein the 5-to 6-membered heteroaromatic ring comprises one, two, or three identical or different heteroatoms, the one, two, or three identical or different heteroatoms being selected from N, O, and S;
preferably, the ring E is selected from a benzene ring, pyridine, pyrimidine, and pyridazine; and
more preferably, Ring E is selected from a benzene ring;
c) R₁ is selected from halogen, hydroxyl, amino, cyano, oxo (=O), C₁ to C₃ alkyl, halogenated C₁ to C₃ alkyl, C₁ to C₃ alkoxy, halogenated C₁ to C₃ alkoxy, C₃ to C₇ cycloalkyl, C₁ to C₃ alkyl-C₁ to C₃ alkoxy, and C₁ to C₃ alkyl-C₃ to C₇ cycloalkyl; and
preferably, R₁ is selected from halogen, methyl, halogenated methyl, and methoxy; or
d) m is selected from 0, 1, 2, 3, or 4; and
preferably, m is selected from 0 or 1.

3. The compound represented by Formula I, or the tautomer, stereoisomer, or pharmaceutically acceptable salt thereof according to claim 1, wherein:
the compound has a structure selected from: 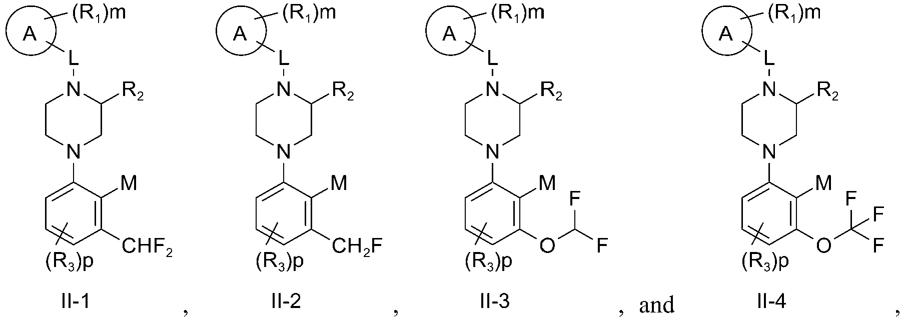
where ring A, M, L, R₁, R₂, R₃, m, and p are as defined in claim 1; and/or
the compound has a structure selected from: 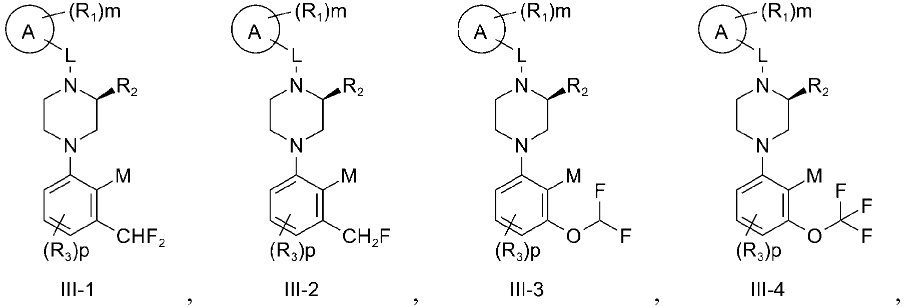 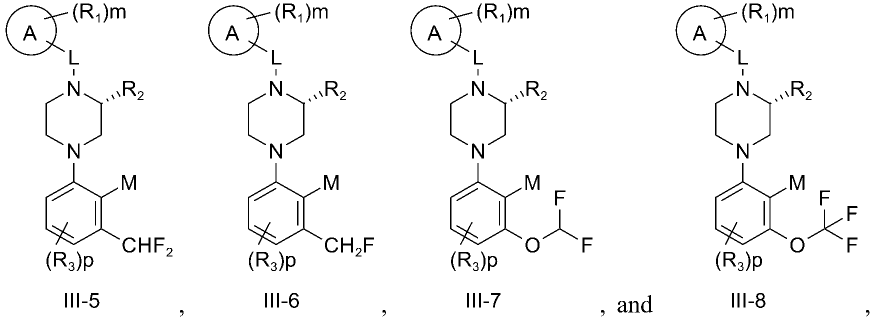 where ring A, M, L, R₁, R₂, R₃, m, and p are as defined in claim 1.

4. The compound represented by Formula I, or the tautomer, stereoisomer, or pharmaceutically acceptable salt thereof according to claim 1, wherein the compound has a structure represented by: 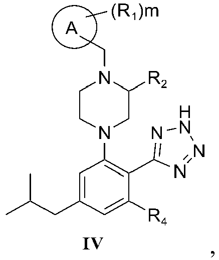 where ring A, R₁, m, R₂, and R₄ are as defined in claim 1.

5. The compound represented by Formula I, or the tautomer, stereoisomer, or pharmaceutically acceptable salt thereof according to claim 1 or 4, wherein the compound has a structure represented by: 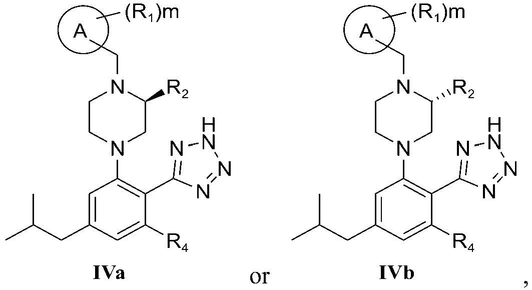 where ring A, R₁, R₂, R₄, and m are as defined in claim 1.

6. The compound represented by Formula I, or the tautomer, stereoisomer, or pharmaceutically acceptable salt thereof according to claim 1 or 4, wherein:
the compound has a structure represented by: 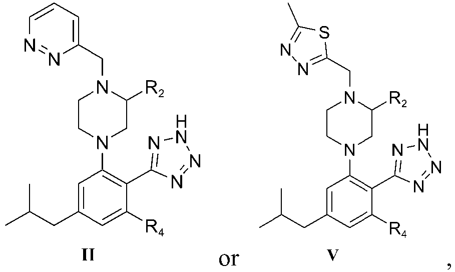 where R₂ and R₄ are as defined in claim 1, and/or
the compound has a structure represented by Formula IIa, Formula IIb, Formula Va, or Formula Vb: 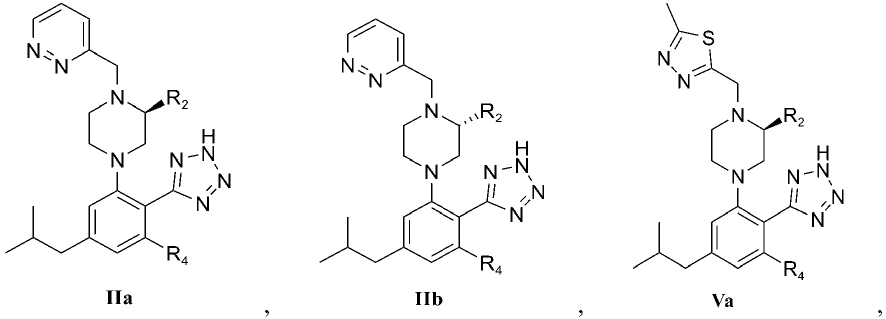 or 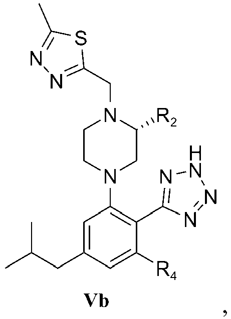
where R₂ and R₄ are as defined in claim 1.

7. The compound represented by Formula I, or the tautomer, stereoisomer, or pharmaceutically acceptable salt thereof according to any one of claims 1, 4, 5, and 6, wherein:
R₄ is selected from -CHF₂, -CH₂F, and -(C₀ to C₃ alkyl)-O-( halogenated C₁ to C₃ alkyl), wherein the -(Co to C₃ alkyl)-O-( halogenated C₁ to C₃ alkyl), if present, is optionally substituted with one to four substituents selected from hydroxyl, amino, C₃ to C₇ heterocycloalkyl, C₁ to C₃ alkyl, C₃ to C₆ cycloalkyl, and C₁ to C₃ alkoxy;
preferably, R₄ is selected from -CHF₂, -CH₂F, and -O-(halogenated C₁ to C₃ alkyl);
preferably, R₄ is selected from -CHF₂, -CH₂F, and -O-(C₁ to C₃ fluoroalkyl);
preferably, R₄ is selected from -CHF₂, -CH₂F, and -O-fluoromethyl; and
preferably, R₄ is selected from -CHF₂, -CH₂F, -O-CHF₂, and -O-CF₃.

8. The compound, or the tautomer, stereoisomer, or pharmaceutically acceptable salt thereof according to any one of claims 1, 3, 4, 5, and 6, wherein:
R₂ is selected from oxo (=O), C₁ to C₃ alkyl, C₁ to C₃ alkoxy, C₃ to C₆ cycloalkyl, a 5- to 6-membered heterocyclic ring, and a benzene ring, wherein the C₁ to C₃ alkyl, the C₁ to C₃ alkoxy, the C₃ to C₆ cycloalkyl, the 5- to 6-membered heterocyclic ring, and the benzene ring are optionally substituted with one, two, three, or four substituents selected from halogen, hydroxyl, cyano, amino, C₁ to C₃ alkyl, C₁ to C₃ alkoxy, and C₃ to C₆ cycloalkyl, and n is selected from 0, 1, 2, 3, 4, 5, 6, 7, or 8;
preferably, R₂ is selected from oxo (=O), C₁ to C₃ alkyl, and halogenated C₁ to C₄ alkyl, and n is selected from 0, 1, 2, 3, and 4; and
more preferably, R₂ is selected from methyl and halogenated methyl, and n is selected from 0 or 1.

9. The compound represented by Formula I, or the tautomer, stereoisomer, or pharmaceutically acceptable salt thereof according to claim 1, satisfying one or more of the following conditions e) to g):
e) R₃ is selected from halogen, C₁ to C₆ alkyl, C₁ to C₆ alkoxy, and C₃ to C₇ cycloalkyl, wherein the C₁ to C₆ alkyl, the C₁ to C₆ alkoxy, and the C₃ to C₇ cycloalkyl are optionally substituted with one to four substituents selected from halogen, hydroxyl, cyano, C₁ to C₆ alkyl, C₁ to C₆ alkoxy, and C₃ to C₇ cycloalkyl, and p is selected from 0, 1, 2, 3, or 4;
preferably, R₃ is selected from F, C₁ to C₅ alkyl, halogenated C₁ to C₅ alkyl, and methyl-cyclopropyl, and p is selected from 1, 2, and 3; and
more preferably, R₃ is selected from F, methyl, trifluoromethyl, isobutyl, fluorinated isobutyl, and methyl-cyclopropyl, and p is selected from 1;
f) M is selected from carboxylic group, -CONHSO₂R₅, -SO₂NHCOR₅, tetrazole, or a tetrazole bioisostere;
preferably, M is selected from carboxylic group, -CONHSO₂R₅, -SO₂NHCOR₅, and tetrazole, R₅ being selected from C₁ to C₃ alkyl and C₃ to C₅ cycloalkyl; and
more preferably, M is selected from tetrazole; or
g) L is absent or selected from methylene, -CH₂CH₂-, -CH(CH₃)-, -CH₂CH₂CH₂-, - CH₂CH(CH₃)-, -O-CH₂-, and -NH-CH₂-;
preferably, L is selected from methylene, -CH₂CH₂-, -CH(CH₃)-, and -O-CH₂-; and
more preferably, L is selected from methylene.

10. The compound represented by Formula I, or the tautomer, stereoisomer, or pharmaceutically acceptable salt thereof according to claim 1, wherein the compound has a structure selected from: 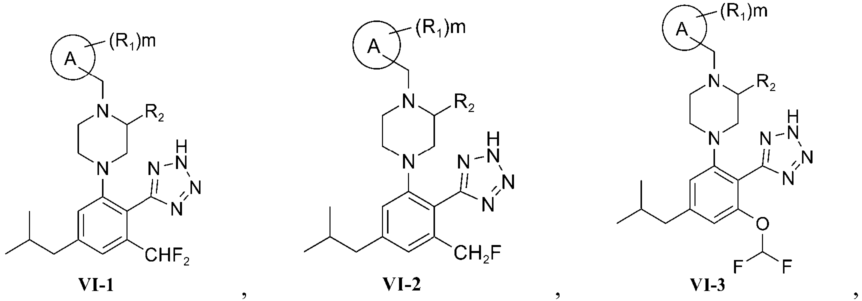 and 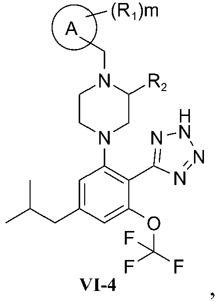 where ring A, R₁, R₂, and m are as defined in claim 1.

11. The compound represented by Formula I, or the tautomer, stereoisomer, or pharmaceutically acceptable salt thereof according to claim 1, wherein the compound has a structure represented by Formula VI-1a, Formula VI-1b, Formula VI-2a, Formula VI-2b, Formula VI-3a, Formula VI-3b, Formula VI-4a, or Formula VI-4b: 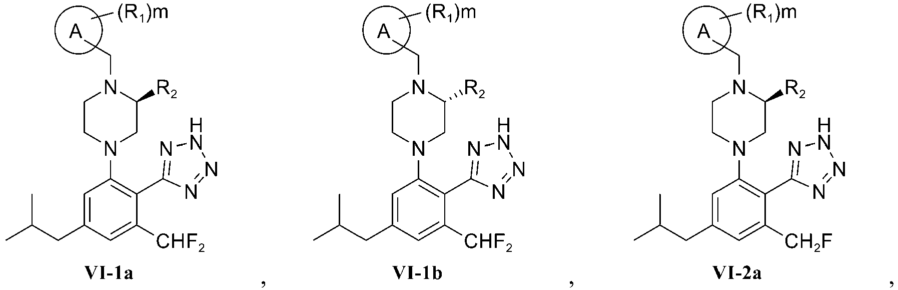 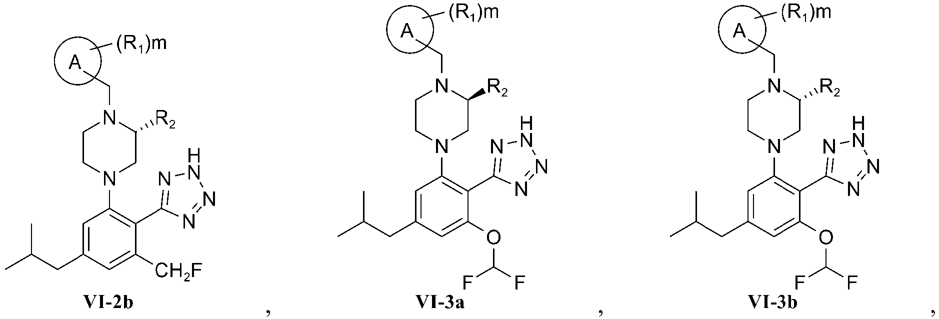 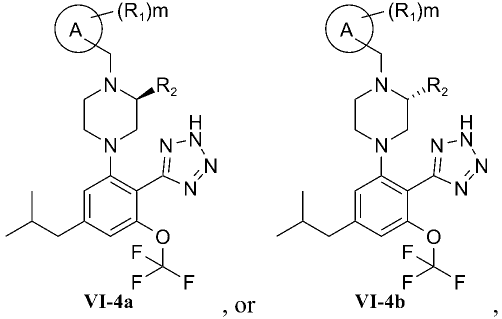 where ring A, R₁, R₂, and m are as defined in claim 1.

12. The compound represented by Formula I, or the tautomer, stereoisomer, or pharmaceutically acceptable salt thereof according to any one of claims 1, 3, 4, 5, 10, and 11, wherein 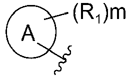 is

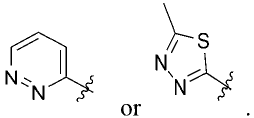

13. The compound, or the tautomer, stereoisomer, or pharmaceutically acceptable salt thereof according to claim 1, wherein the compound comprises:

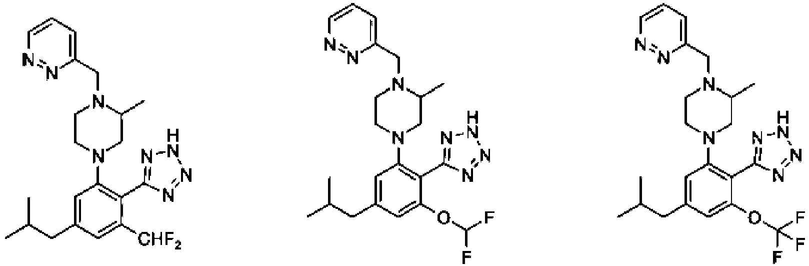

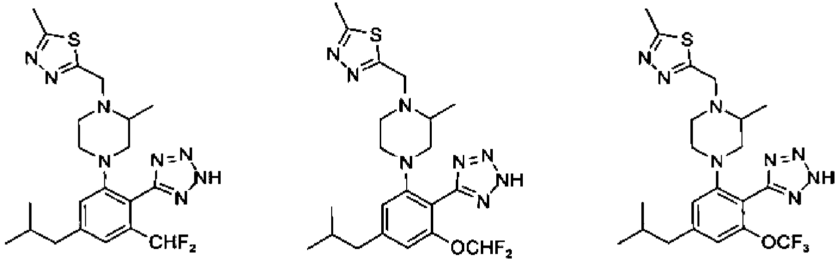

14. The compound represented by Formula I, or the tautomer, stereoisomer, or pharmaceutically acceptable salt thereof according to claim 1, wherein the compound comprises:

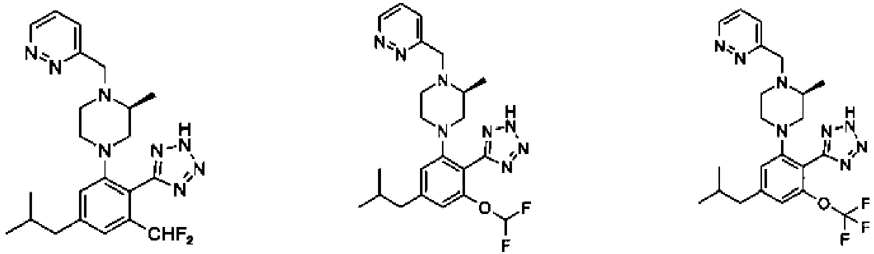

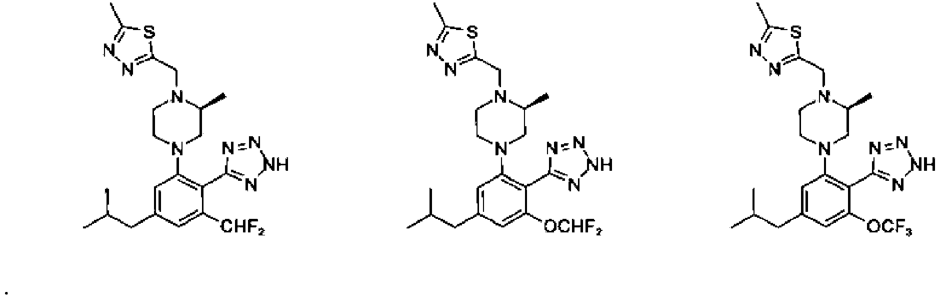

15. A pharmaceutical composition, comprising:
the compound, or the tautomer, stereoisomer, or pharmaceutically acceptable salt thereof according to any one of claims 1 to 14; and
a pharmaceutically acceptable carrier.

16. Use of the compound, or the tautomer, stereoisomer, or pharmaceutically acceptable salt thereof according to any one of claims 1 to 14, or the pharmaceutical composition according to claim 15, the use comprising:
acting as an AT2R antagonist; and/or
preventing and/or treating an AT2R-mediated disease; and/or
preventing and/or treating a disease where a reduced effect of Ang II is desired or required; and/or
preparing a medicament, pharmaceutical composition, or formulation as an AT2R antagonist; and/or
preparing a medicament, pharmaceutical composition, or formulation for preventing and/or treating a disease where AT2R is expressed and an inhibition of AT2R is desired or necessary.

17. The use according to claim 16, wherein the disease is neuropathy or neuropathic pain.

18. The use according to claim 17, wherein the disease is primary neuropathy, secondary neuropathy, peripheral neuropathy, neuropathy caused by mechanical nerve injury or biochemical nerve injury, postherpetic neuralgia, diabetic neuropathic pain, or a diabetes-related neuropathic disease.

19. A method for treating a disease, the method comprising:
administering a therapeutically effective dose of the compound, or the tautomer, stereoisomer, or pharmaceutically acceptable salt thereof according to any one of claims 1 to 14, or the pharmaceutical composition according to claim 15 to a subject suffering from or susceptible to the disease,
wherein the disease is:
an AT2R-mediated disease; and/or
a disease where a reduced effect of Ang II is desired or necessary; and/or
a disease where AT2R is expressed and an inhibition of AT2R is desired or necessary.

20. The method according to claim 19, wherein:
the disease is neuropathy or neuropathic pain; and/or
the disease is primary neuropathy, secondary neuropathy, peripheral neuropathy, neuropathy caused by mechanical nerve injury or biochemical nerve injury, postherpetic neuralgia, diabetic neuropathic pain, or a diabetes-related neuropathic disease.
